# EUROPEAN PATENT SPECIFICATION

(11) **EP 2 875 360 B1**
(45) Date of publication and mention of the grant of the patent: **08.08.2018**
(21) Application number: 13820192.6
(22) Date of filing: 22.07.2013
(51) Int. Cl.: C12Q 1/6883, G01N 33/50

(54) **IN VITRO ASSAY FOR PREDICTING RENAL PROXIMAL TUBULAR CELL TOXICITY**
IN-VITRO-TEST ZUR VORHERSAGE VON RENALER PROXIMALER TUBULÄRER TOXIZITÄT
DOSAGE IN VITRO PERMETTANT DE PRÉDIRE LA CYTOTOXICITÉ TUBULAIRE PROXIMALE RÉNALE

(30) Priority: 20.07.2012 US 201261674018 P; 25.07.2012 US 201261675680 P
(43) Date of publication of application: 27.05.2015
(73) Proprietor: Agency for Science, Technology and Research, Singapore 138632 (SG)
(72) Inventor: ZINK, Daniele, Singapore 138669 (SG); LI, Yao, Singapore 138669 (SG)
(74) Representative: AWA Sweden AB
(86) International application number: PCT/IB2013/001944
(87) International publication number: WO 2014/013334

(56) References cited:
- US-A1- 2009 220 982
- US-A1- 2011 236 874
- YAO LI ET AL: "An in vitro method for the prediction of renal proximal tubular toxicity in humans", TOXICOLOGY RESEARCH, vol. 2, no. 5, 1 January 2013 (2013-01-01) , page 352, XP055228144, ISSN: 2045-452X, DOI: 10.1039/c3tx50042j
- ASTASHKINA AI ET AL.: 'A 3-D organoid kidney culture model engineered for high- throughput nephrotoxicity assays.' BIOMATERIALS vol. 33, no. 18, June 2012, pages 4700 - 4711, XP028411045
- MILLER RP ET AL.: 'Mechanisms of Cisplatin nephrotoxicity.' TOXINS vol. 2, no. 11, November 2010, BASEL, pages 2490 - 2518, XP055185352
- KAWAKAMI T ET AL.: 'Indoxyl sulfate inhibits proliferation of human proximal tubular cells via endoplasmic reticulum stress.' AM J PHYSIOL RENAL PHYSIOL. vol. 299, no. 3, September 2010, pages F568 - F576, XP055185353
- BAER PC ET AL.: 'Effects of mycophenolic acid on IL-6 expression of human renal proximal and distal tubular cells in vitro.' NEPHROL DIAL TRANSPLANT. vol. 19, no. 1, January 2004, pages 47 - 52, XP055185354
- FUENTE MORA C ET AL.: 'Differentiation of podocyte and proximal tubule-like cells from a mouse kidney-derived stem cell line.' STEM CELLS DEV. vol. 21, no. 2, 20 January 2012, pages 296 - 307, XP055185356

## Description

### FIELD OF THE INVENTION

The present invention relates to *in vitro* assay methods for predicting the toxicity of a compound for renal proximal tubular cells, including predicting toxicity *in vivo.*

### BACKGROUND OF THE INVENTION

The kidney is one of the major target organs for drug-induced toxicity. Nephrotoxic drugs and chemicals can induce acute kidney injury (AKI), or chronic kidney disease and subsequently end stage renal disease (ESRD) (1-3). AKI and ESRD patients have increased morbidity and mortality and depend on dialysis (1, 4, 5). About 5% of all hospitalized patients and ∼20%-30% of ICU patients develop AKI, and ∼20%-25% of these cases are due to nephrotoxic drugs (2-4). When alternative and new drugs become available their nephrotoxic potential is often underestimated (6), which leads again to clinical complications, as in case of COX2 inhibitors (7).

Typically, nephrotoxicity is detected only late during drug development and accounts for 2% of drug attrition during pre-clinical studies and 19% in phase 3 (8). Also, due to the large functional reserve of the kidney nephrotoxic effects often become obvious only after regulatory approval. A recent example is tenofovir, which injures the renal proximal tubules (9, 10). Together, the problems outlined above are associated with increased risks for patients and subjects enrolled in clinical trials as well as substantial costs for the health care system and the pharmaceutical industry.

One major problem is the lack of pre-clinical models with high predictability. The predictability of animal models is compromised by interspecies variability, and there are other problems such as high costs and low throughput. Further, legislation changes in the EU (REACH and the 7^{th} Amendment of the Cosmetics Directive) and new initiatives in the USA (ToxCast and Tox21) have increased the interest in *in vitro* models. Regulatory accepted or validated *in vitro* models for the prediction of nephrotoxicity in humans are currently not available. Major difficulties are related to the identification of appropriate cell types and endpoints (11-13).

In the kidney the cells of the renal proximal tubule (PT) are a major target for drug-induced toxicity due to their roles in glomerular filtrate concentration and the transport of drugs and organic compounds (2, 3). PT-derived cell lines, such as the human and porcine cell lines HK-2 (human kidney-2) and LLC-PK1 (Lewis lung cancer-porcine kidney 1), have been frequently applied in *in vitro* nephrotoxicology. However, immortalized cells are less sensitive than human primary renal proximal tubular cells (HPTC) (14) and insensitive to well-known nephrotoxicants (13), which is due to do functional changes and changes in drug transporter expression associated with immortalization (15-17). Further, endpoints that are associated with general cytotoxicity, such as cell death, metabolic activity or ATP depletion, are not useful for addressing organ-specific toxicity. A recent study measuring ATP-depletion in liver-, kidney PT- and heart-derived cell lines treated with hepatotoxic, nephrotoxic and cardiotoxic compounds found that the majority of compounds had similar effects in all three cell lines (18).

The European and US regulatory agencies in charge of the validation and acceptance of alternative methods (European Centre for the Validation of Alternative Methods (ECVAM) and the National Toxicology Program Interagency Center for the Evaluation of Alternative Toxicological Methods/Interagency Coordinating Committee on the Validation of Alternative Methods (NICEATM/ICCVAM)) are currently not involved in any activities on the validation of methods for *in vitro* nephrotoxicology. The ECVAM has funded one pre-validation study (19) which used 15 drugs. Other models for *in vitro* nephrotoxicology that have been developed since then during the last 10 years (20-24) have been tested with limited numbers of drugs and are of unclear predictability. A recently developed high-throughput mitochondrial nephrotoxicant assay is based on rabbit cells (25); the use of non-human animal model may raises issues concerning interspecies variability. This applies also to a model employing PT freshly isolated from murine kidneys (23, 24).

US 2009/220982 relates to an in vitro assay for determining the nephrotoxicity of a compound. These assays correlate well with in vivo nephrotoxicity and may be adapted to screen for nephroprotectant compounds, including those that protect cells and animals from the nephrotoxic effects of aminoglycoside antibiotics.

### SUMMARY OF THE INVENTION

The methods of the present invention relate to *in vitro* assays for predicting renal proximal tubule toxicity of a compound, and may include predicting *in vivo* toxicity. The methods use expression levels of IL-6 and/or IL-8 in renal proximal tubular cells to assess toxicity of a test compound.

The *in vitro* assay may be performed using human cells, and thus may provide a model for the prediction of renal proximal tubular toxicity in humans.

The predictability of the model may be, in some instances, fairly high, for example in the range of approximately 76%-85%.

When used as a model for human disease, the methods may allow prediction of renal proximal tubular toxicity at an early pre-clinical stage during drug development. Such prediction ability could be important for developing safer drugs, and early detection of nephrotoxicity could also help to save substantial costs during drug development.

In one aspect, the present invention provides an *in vitro* method for screening renal proximal tubular cell toxicity of a compound. The method comprises contacting a test compound with a test population of renal proximal tubular cells; and determining the expression level of an interleukin in the test population, the interleukin being interleukin-6 (IL-6) or interleukin-8 (IL-8), or both. The expression levels of the interleukin in the test population being greater than expression levels in a control population of renal proximal tubular cells not contacted with the test compound is indicative that the test compound is toxic for renal proximal tubular cells.

Determining of the expression level of the interleukin may comprise determining the levels of mRNA encoding the interleukin, and may comprise one or more of quantitative PCR techniques, northern blot techniques, microarray techniques, TRAC techniques, fluorescent label techniques and phosphorimaging techniques.

Determining of the expression level of the interleukin may comprise determining the level of secreted interleukin protein, and may comprise one or more of ELISA techniques, biosensor techniques, electrochemical detection techniques, immunoblotting techniques, cytometric bead array techniques, fluorescent label techniques, and receptor binding techniques.

Determining of the expression level of the interleukin may comprise detecting levels of a reporter gene expressed under control of IL-6 or IL-8 regulation.

The ratio of expression levels of the interleukin in the test population to expression levels of the interleukin in the control population being about 1.5 or greater, or about 3.5 or greater, may be indicative that the test compound is toxic for renal proximal tubular cells.

The renal proximal tubular cells may be derived from somatic cells or from stem cells. In some embodiments, the renal proximal tubular cells are derived from somatic cells and may be primary cells or cells from a stable cell line, including for example human primary renal proximal tubular cells, HK-2 cells, or LLC-PK1 cells.

The renal proximal tubular cells may be derived from stem cells and may be differentiated from embryonic stem cells, mesenchymal stem cells, or induced pluriopotent stem cells.

In some embodiments, the proximal tubular cells are human renal proximal tubular cells. In some embodiments, the proximal tubular cells are non-human proximal tubular cells.

The contacting may be performed over a period of time of about 8 hours or longer. The contacting may be repeated one or more times in a period of from about 3 to about 14 days.

The contacting may comprise adding the test compound to the test population of renal proximal tubular cells at a concentration of about 0.001 to about 1000 µg/ml.

The test population may be a confluent monolayer, a subconfluent monolayer, a confluent epithelium, an organoid culture, a confluent 2D culture, an *in vitro* tubule, a 3D organoid culture or a 3D culture cultivated under static or microfluidic conditions.

Other aspects and features of the present invention will become apparent to those of ordinary skill in the art upon review of the following description of specific embodiments of the invention in conjunction with the accompanying figures.

### BRIEF DESCRIPTION OF THE DRAWINGS

The figures, which illustrate, by way of example only, embodiments of the present invention, are as follows.

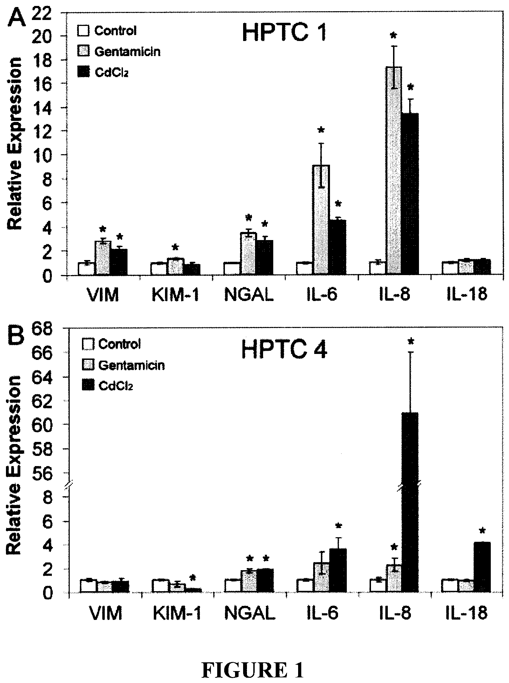

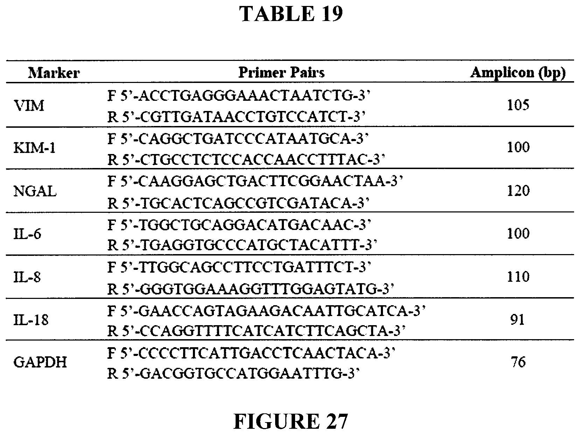

**Figure 1****.** Marker gene expression in response to nephrotoxins. Two different batches of HPTC (1 and 4) derived from different donors were treated with 2.5 mg/ml gentamicin (light grey bars) and 10 µg/ml CdCl₂ (dark grey bars; vehicle control: white bars). High doses of these nephrotoxins were applied in order to exclude that a lack of response was due to a dosing problem. The relative expression levels of the marker genes indicated on the x-axis were determined by qPCR (for primers see Table 19 (Figure 27). The bars show the mean fold expression compared to the vehicle control +/- standard deviation (s.d.; n = 3). The means of the vehicle controls from each experiment were set to 1. Significant differences (P < 0.05) in comparison to the vehicle control are indicated by asterisks.

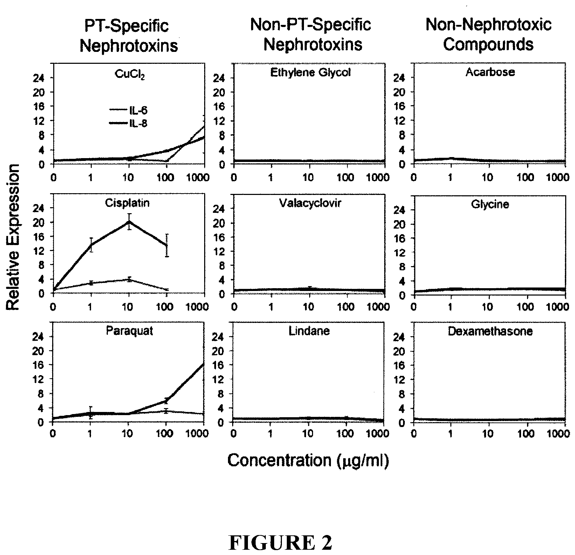

**Figure 2****.** Dose-response curves. HPTC 1 were exposed to proximal tubular (PT)-specific nephrotoxins (group 1, left-hand panels), nephrotoxins that are not toxic for the proximal tubule (group 2, middle) or non-nephrotoxic compounds (group 3, righthand panels) at the concentrations indicated on the x-axis (note logarithmic scale). The figure shows the expression levels of IL-6 (grey graphs) and IL-8 (black graphs) relative to the expression levels of the vehicle control (mean +/- s.d.). In the case of cisplatin, massive cell death occurred at the highest concentration tested.

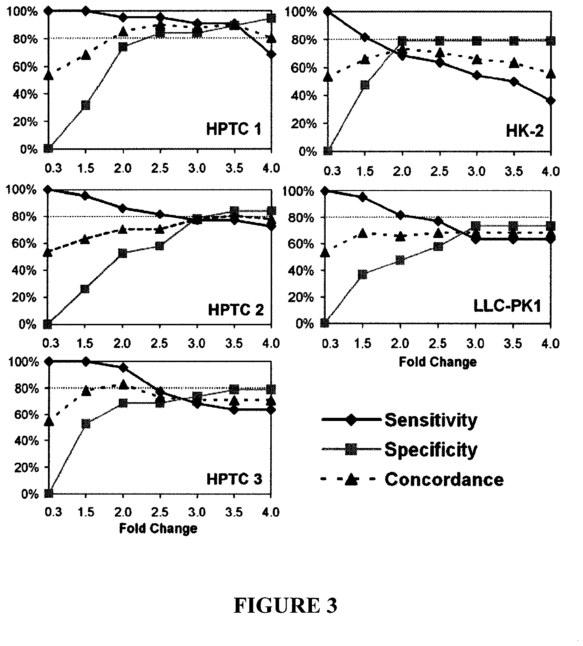

**Figure 3****.** Sensitivity, specificity and overall concordance with clinical data. The figure displays graphically the percentages for sensitivity and specificity shown in Table 5. In addition, the figure shows the overall concordance with clinical data. Calculations were performed separately for the three different batches of HPTC as well as for HK-2 and LLC-PK1 cells. Thresholds (x axis) ranged from 0.3-4.0. The 80% value is indicated by a dotted line to facilitate comparisons.

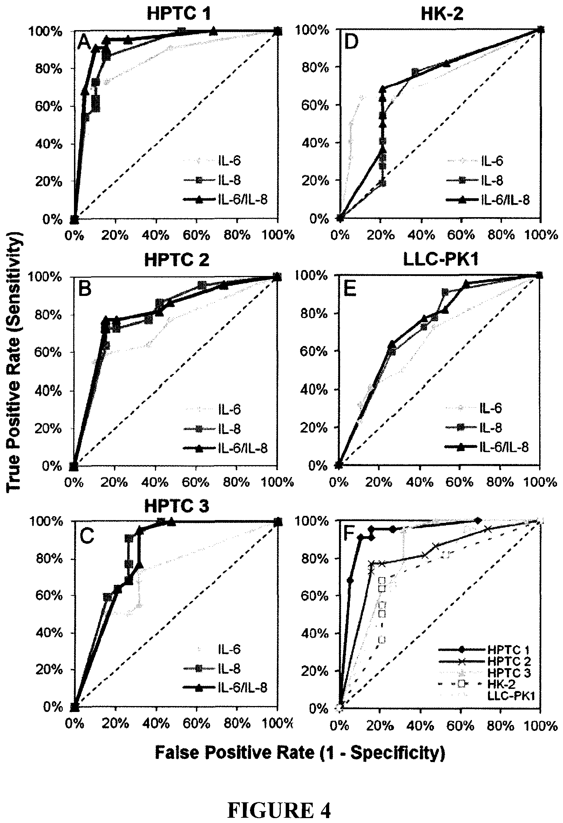

**Figure 4****.** Receiver operating characteristic (ROC) curves. For each cell batch/type the ROC curves were calculated for each single marker or the combination of both markers. The respective area under the curve (AUC) values are summarized in Table 6. For comparison panel F displays simultaneously the ROC curves (combination of both markers) obtained for all different cell batches/types tested.

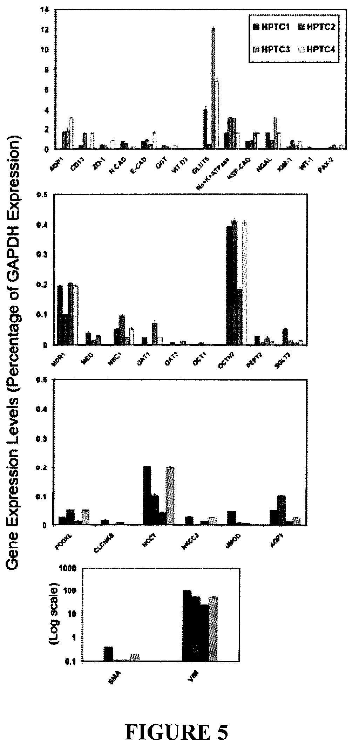

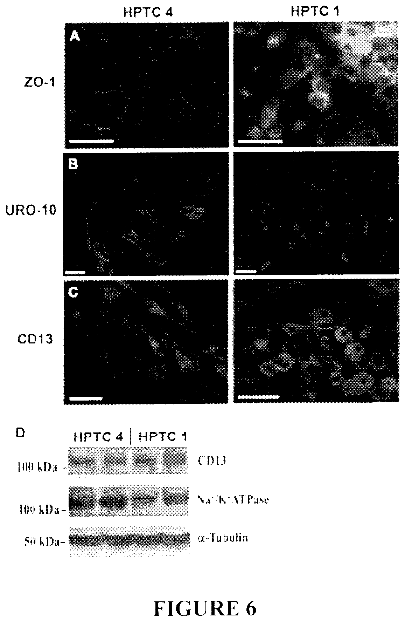

**Figure 5****.** Marker gene expression determined by qPCR in four different batches of HPTC (1-4). The relative expression levels of the 31 genes indicated (x-axis) are displayed as percentage of GAPDH expression (y-axis). The bars show the mean +/standard deviation (s.d.; n = 3). Due to differences in expression levels the scales on the γ-axes of the different diagrams are different and a log scale has been used with respect to (alpha smooth muscle actin (SMA) and vimentin (VIM; bottom). In addition to these 2 markers for trans- and dedifferentiation, the following epithelial, HPTC-specific, renal, and renal injury-specific markers were included: aquaporin-1 (AQP1), aminopeptidase N (CD13), zonula occludens 1 (ZO-1), N-cadherin (N-CAD), E-cadherin (E-CAD), gamma-glutamyl transferase (GGT), 25-hydroxyvitamin D3 1alpha-hydroxylase (VIT D3), glucose transporter 5 (GLUT5), Na+/K+ ATPase, kidney-specific cadherin (KSP-CAD), neutrophil gelatinase-associated lipocalin (NGAL), kidney injury molecule-1 (KIM-1), Wilms' tumor gene 1 (WT1), paired box gene 2 (PAX2), multidrug resistance gene 1 (MDR1), megalin (MEG), Na+HCO3- co-transporter 1 (NBC1), organic anion transporter 1 (OAT1), OAT3, organic cation transporter 1 (OCT1), organic cation/carnitine transporter 2 (OCTN2), proton-coupled peptide transporter 2 (PEPT2), sodium-dependent glucose co-transporter 2 (SGLT2). In addition, the following markers were included that were specific for other parts of the nephron: podocalyxin-like (PODXL, glomerulus), chloride channel Kb (CLCNKB, distal nephron), thiazide-sensitive sodium-chloride co-transporter (NCCT, distal tubule), Na+/K+/2Cl- co-transporter (NKCC2, thick ascending loop of Henle), uromodulin (UMOD, thick ascending loop of Henle and distal convoluted tubules) and AQP3 (collecting duct). For primers see references 27 and 28 and Figure 27 (Table 19). HPTC were analyzed at passage (P) 1. The results shown here are in agreement with our previous results obtained with HPTC cultivated up to P 5 (see reference 27). For instance, in agreement with our previous results obtained with HPTC at higher passage numbers OAT3 and OCT1 were expressed at extremely low levels and HPTC expressed some markers that are *in vivo* expressed in other parts of the nephron/kidney (NCCT and AQP 3) at relatively high levels. Also the finding that VIM is expressed at high levels *in vitro* is in agreement with previous results (references 27 and 46). Some HPTC-specific markers, like GGT, CD13 and ZO-1 are expressed at low levels (∼ 0.4% of GAPDH expression in HPTC 1 obtained from ATCC). In case of GGT quantitative PCR data have been published previously (A. Saito, K. Sawada and S. Fujimura, Hemodial Int, 2011, 15, 183-192.), which are in agreement with the low expression levels observed here. Nevertheless, GGT is functional in HPTC obtained from ATCC (references 27 and 28) and CD13 as well as ZO-1 can be detected by immunoblotting and immunostaining (see Figure 6).
**Figure 6****.** Marker expression determined by immunostaining and immunoblotting in HPTC 1 and 4. (A) ZO-1, (B) URO-10 and (C) CD13 were detected by immunostaining. The tight junctional protein ZO-1 is organized into the characteristic chicken wire-like patterns indicating extensive tight junction formation. Scale bars: 50 µm (A and C) and 100 µm (B). (D) CD13 and the α-subunit of the Na+/K+ ATPase were detected by immunoblotting. α-tubulin was used as loading control. The positions of the 100 kDa and 50 kDa size marker bands are shown on the left. Antibodies and immunoblotting and immunstaining procedures are described in reference 27.

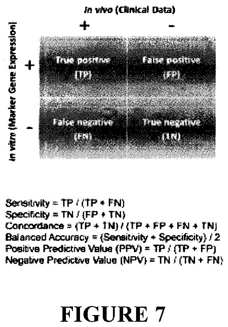

**Figure 7****.** Terms and definitions. The matrix illustrates the definitions of true positives (TP), false positives (FP), false negatives (FN) and true negatives (TN) in relation to positive (+) and negative (-) clinical and *in vitro* results. Definitions of performance metrics are provided.

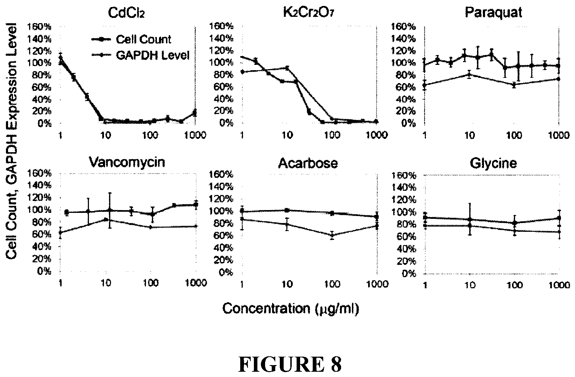

**Figure 8****.** GAPDH levels and cell numbers. GAPDH levels (blue graphs) were determined by qPCR and cell numbers (red graphs) were counted by HCS. The graphs show the values (mean +/- s.d., n = 3) determined at different concentrations (x-axis) of the indicated compounds. All values are expressed as percentage of the vehicle control. The results were obtained with HPTC 1.

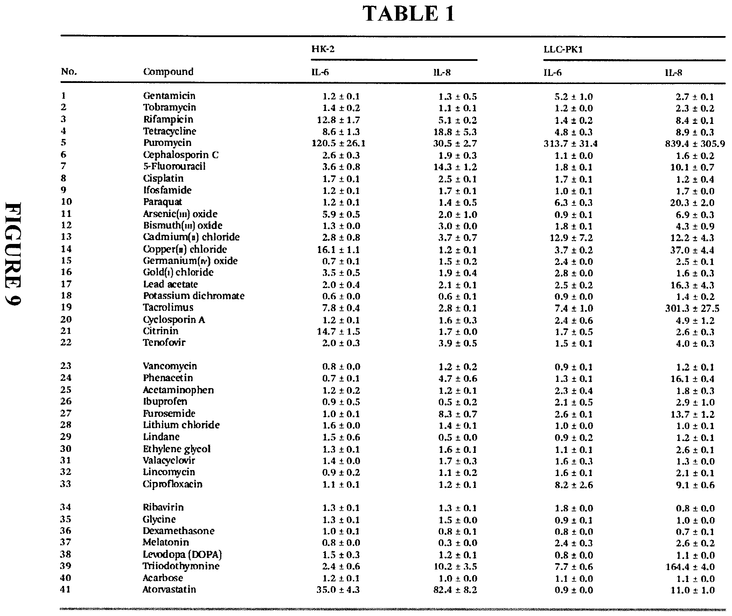

**Figure 9****. (Table 1)** Test compounds and highest expression levels of IL-6 and IL-8 in HK-2 and LLC-PK1 cells. The table lists the 41 test compounds, which were divided into three groups. Group 1 (compounds 1-22) represents nephrotoxins that directly damage the PT. Group 2 (compounds 23-33) comprises nephrotoxins that do not directly damage the PT and injure the kidney by different mechanisms. Group 3 (compounds 34-41) represents non-nephrotoxic compounds. HK-2 and LLC-PK1 cells were exposed to these compounds at concentrations ranging from 1 µg ml⁻¹ to 1000 µg ml⁻¹. The table lists the highest expression levels of IL-6 and IL-8 that were observed at any given concentration of a drug within this range. The numbers show the mean fold expression ±s.d. (n = 3) relative to the vehicle control. The highest expression levels shown here in Table 1 are highlighted in Tables 15-18, which display in detail the expression levels obtained at all the drug concentrations tested.

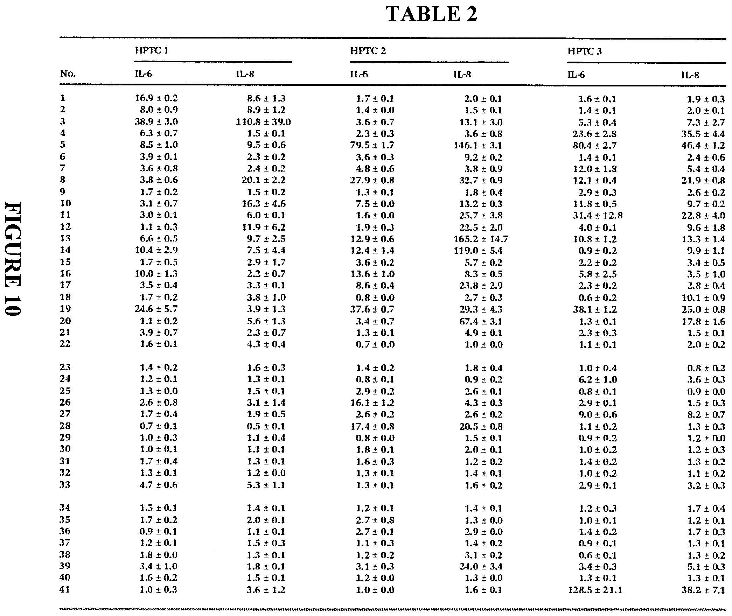

**Figure 10****. (Table 2)** Highest expression levels of IL-6 and IL-8 in HPTC. Three different batches of HPTC derived from different donors were exposed to the 41 test compounds at concentrations ranging from 1 µg ml⁻¹ to 1000 µg ml⁻¹. The table lists the highest expression levels of IL-6 and IL-8 that were observed at any given concentration of a drug within this range. The numbers show the mean fold expression ±s.d. (n = 3) relative to the vehicle control. The highest expression levels shown here in Table 2 are highlighted in the Tables 9-14, which display in detail the expression levels obtained at all the drug concentrations tested.

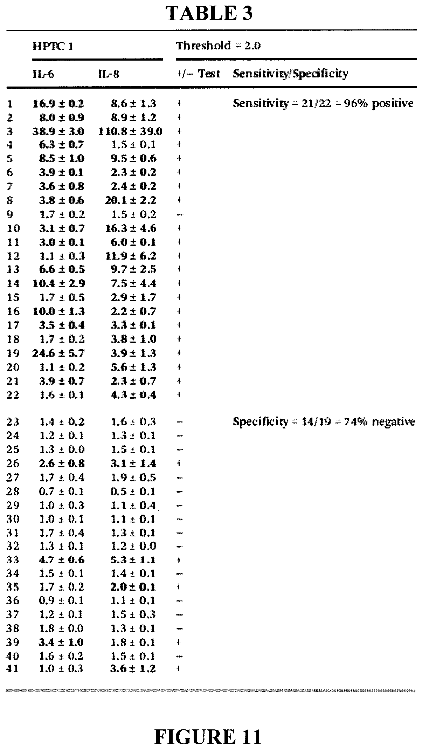

**Figure 11****. (Table 3)** Example for the thresholding procedure, determination of positive and negative results and calculation of sensitivity and specificity. The HPTC 1data set shown here is identical with the respective data set in Table 2. A threshold of 2.0 was applied. If for a given drug the expression value of at least one marker gene was equal to or above the threshold a result was classified as positive (+). If for a given drug the expression levels of both marker genes were below the threshold the result was classified as negative (-). Based on these positive and negative results the sensitivity and specificity were calculated. Sensitivity was defined as the number of positive group 1 drugs (TP) divided by the total number of 22 group 1 drugs. Specificity was defined as the number of negative group 2 and 3 drugs (TN) divided by the total number of 19 group 2 and 3 drugs.

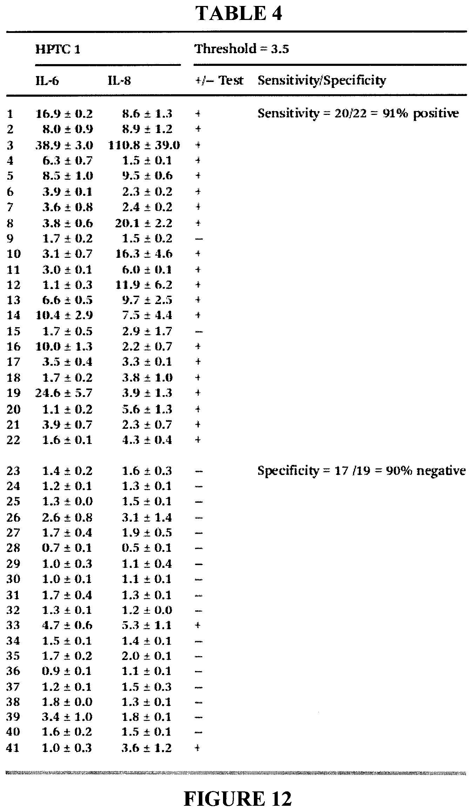

**Figure 12****. (Table 4)** Example for the thresholding procedure, determination of positive and negative results and calculation of sensitivity and specificity. The HPTC 1 data set is identical with the data set shown in Figure 11 (Table 3). Here in Table 4 a different threshold level of 3.5 was applied to this data set. For detailed explanations see the legend of Figure 11 (Table 3).

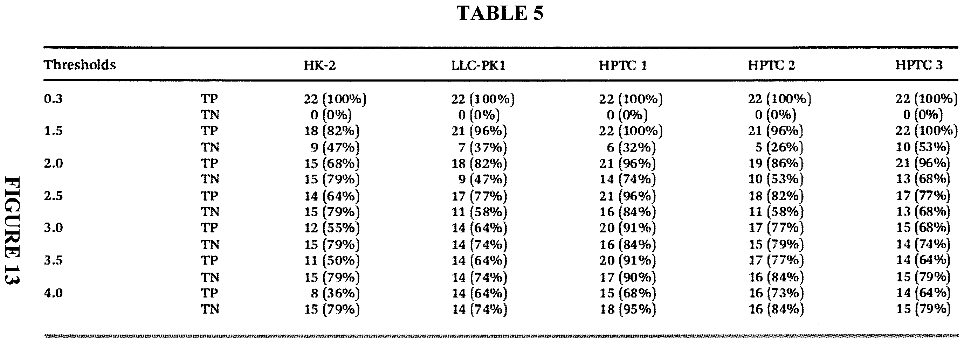

**Figure 13****. (Table 5)** Determination of true positives (TP), true negatives (TN), sensitivity and specificity. TP were defined as true PT-specific nephrotoxins (22 drugs, group 1) that were correctly detected as positives by this assay. TN were defined as non-PT-specific nephrotoxins and non-nephrotoxic drugs (19 drugs; groups 2 and 3) that remained negative in our assay. How positive and negative assay results were obtained at different threshold levels is shown in Tables 3 and 4. TP and TN were determined at the indicated threshold levels ranging from 0.3 to 4.0 and the numbers are displayed. From these numbers, the percentages of sensitivity (TP/total number of group 1 drugs × 100%) and specificity (TN/total number of group 2 + 3 drugs × 100%) were calculated. The percentages of sensitivity and specificity are displayed in brackets together with the respective numbers of TP (sensitivity) and TN (specificity). The numbers of TP and TN and respective percentages of sensitivity and specificity were determined for all cell types and batches based on the results from all 41 drugs. The percentages of sensitivity and specificity shown here are graphically displayed in Figure 3.

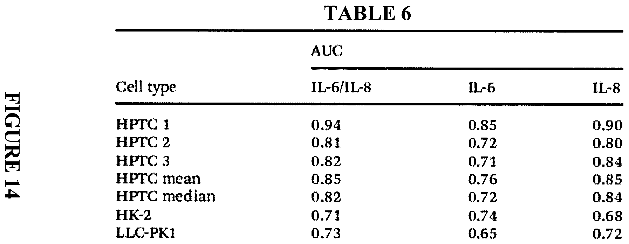

**Figure 14****. (Table 6)** AUC values. The table provides the AUC values of the ROC curves (Figure 4) for every cell batch/type tested. For HPTC also the mean and median values are shown. AUC values were determined separately for either IL-6 or IL-8 or the combination of these two markers. AUC values >0.5 represent a predictive model that is better than chance.

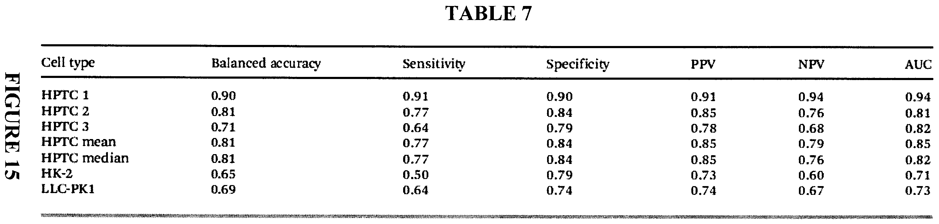

**Figure 15****. (Table 7)** Performance metrics. The table summarizes the values for the following performance metrics: balanced accuracy (defined as the average between sensitivity and specificity), sensitivity, specificity, positive predictive value (PPV), negative predictive value (NPV) and AUC. In the case of sensitivity, specificity, PPV and NPV the values obtained at a threshold value of 3.5 (see Figure 3) are displayed. With respect to the AUC values the results obtained with a combination of both markers are provided. These values are identical with those in Figure 14 (Table 6) and are displayed here again for completeness.

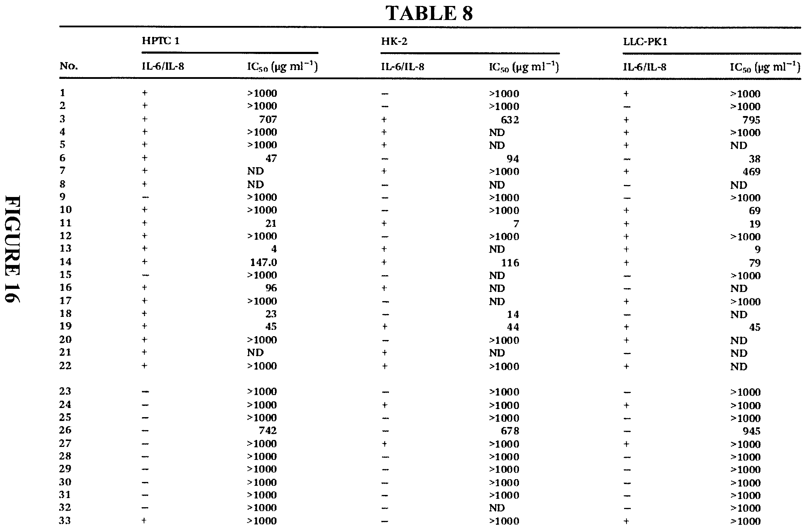

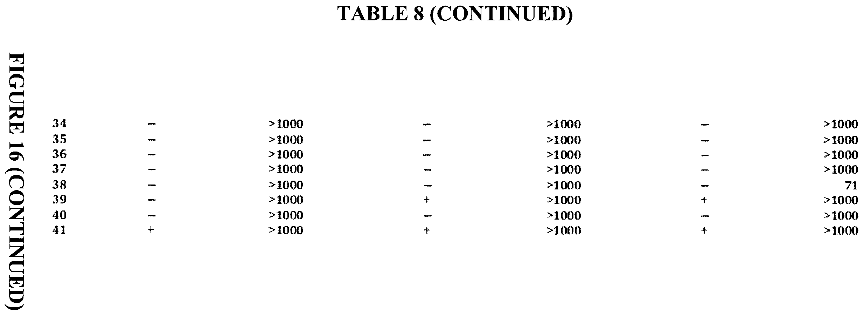

**Figure 16****. (Table 8)** Comparison of drug effects on IL-6/IL-8 expression and cell numbers. HPTC 1, HK-2 and LLC-PK1 cells were exposed to the 41 test compounds. Data on IL-6/IL-8 expression were based on previous results (Tables 1 and 2). A result was defined as positive (+) when expression of at least one marker showed at any concentration an increase of 3.5-fold or above. If marker expression values remained below 3.5-fold the result was classified as negative (-). IC₅₀ values were calculated based on cell numbers determined by HCS. A value of >1000 µg ml⁻¹ was assigned if cell viability was >50% at the highest concentration of a compound (1000 µg ml⁻¹). Cell numbers were not determined (ND) in some cases. (Table 8 includes SEQ ID NOs.: 5-18.)

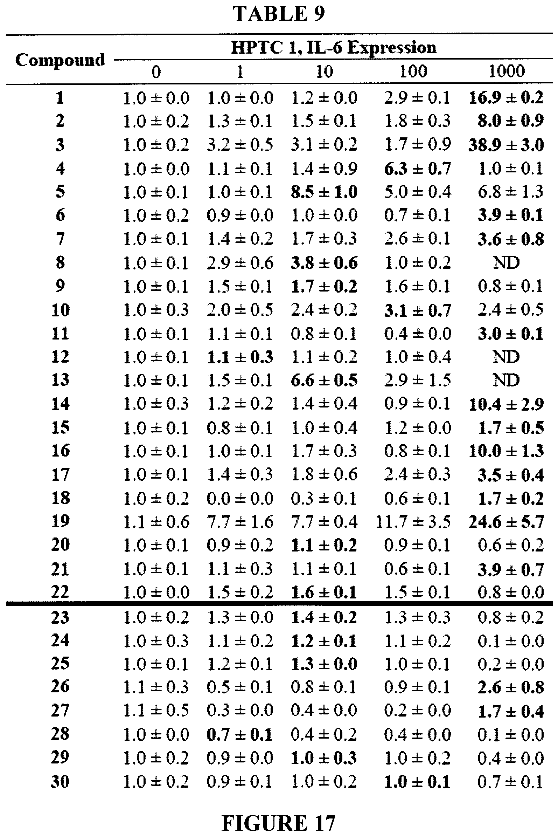

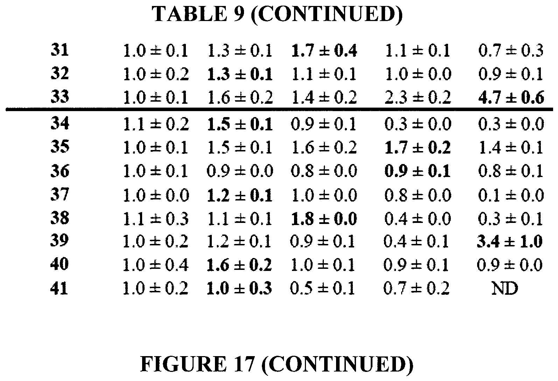

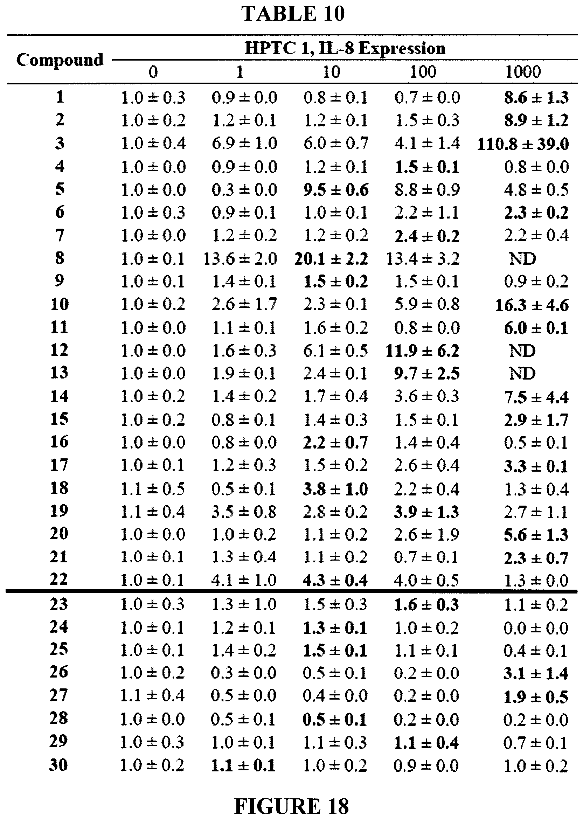

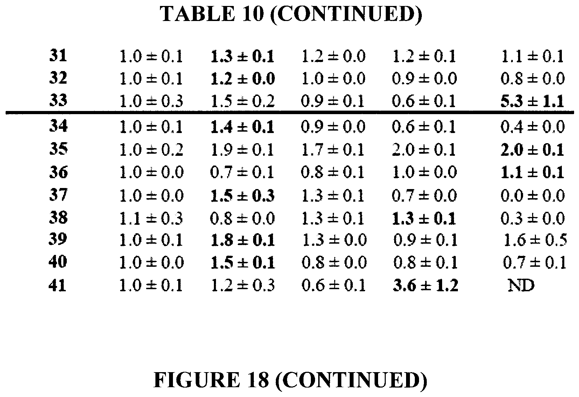

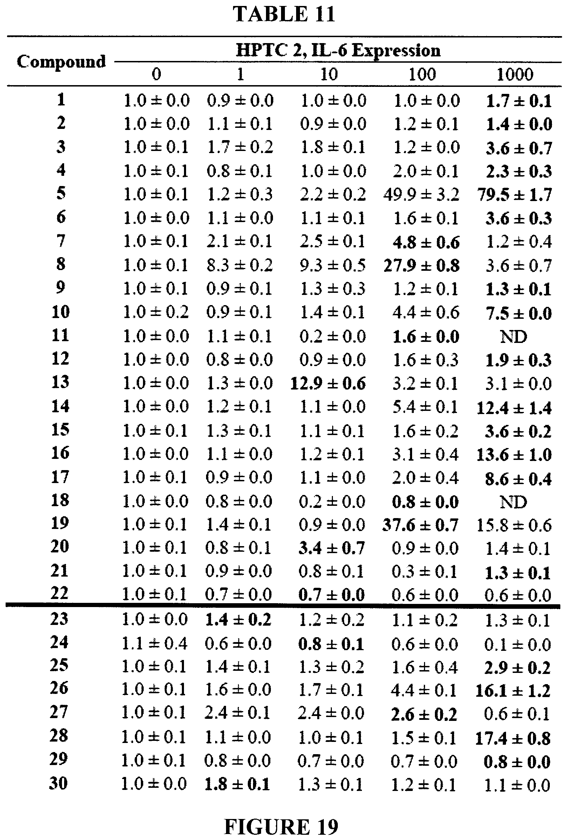

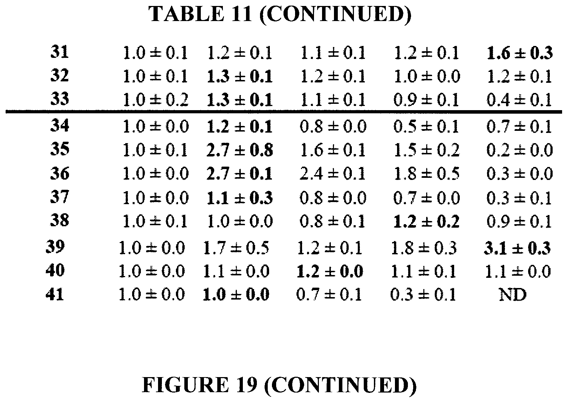

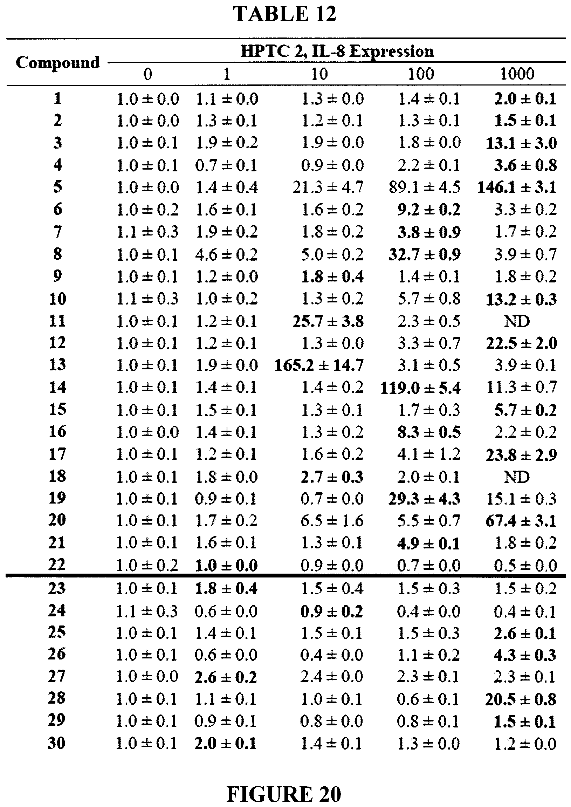

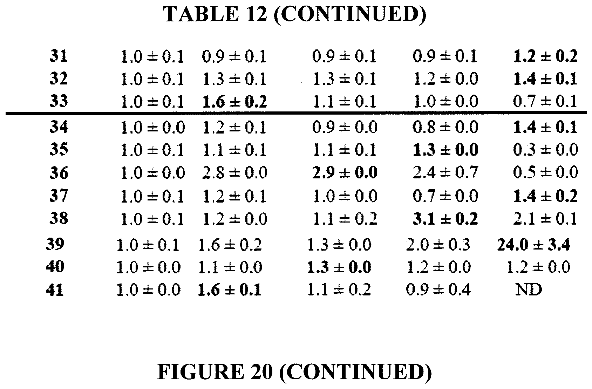

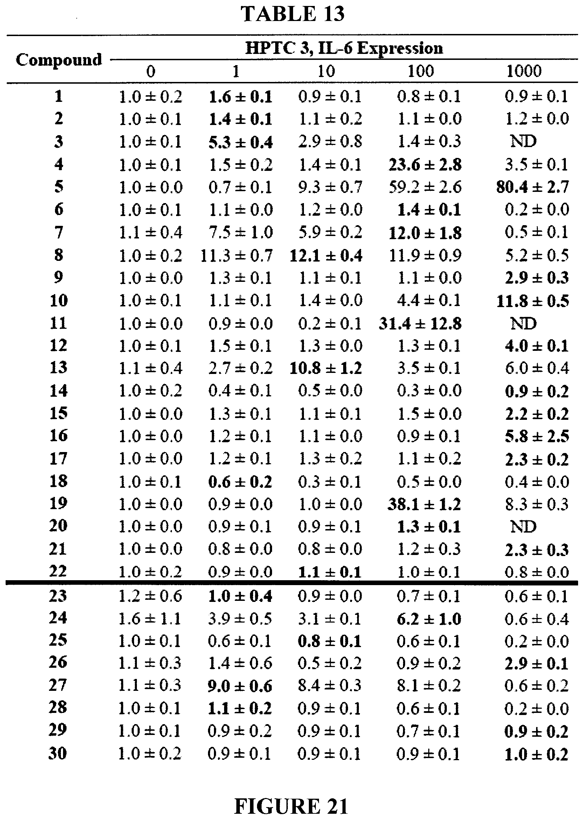

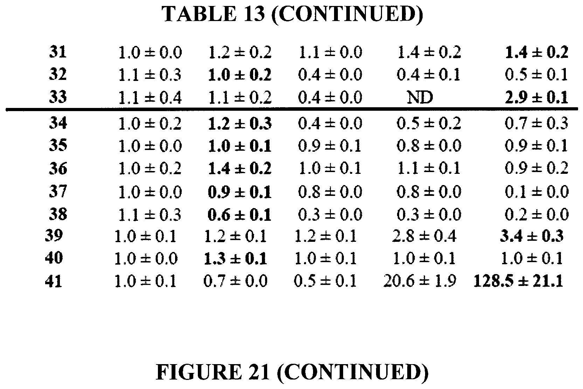

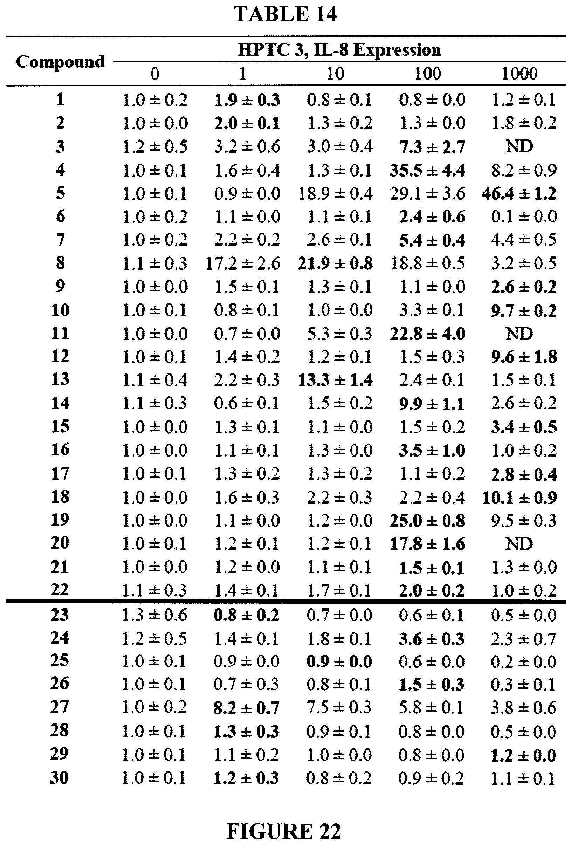

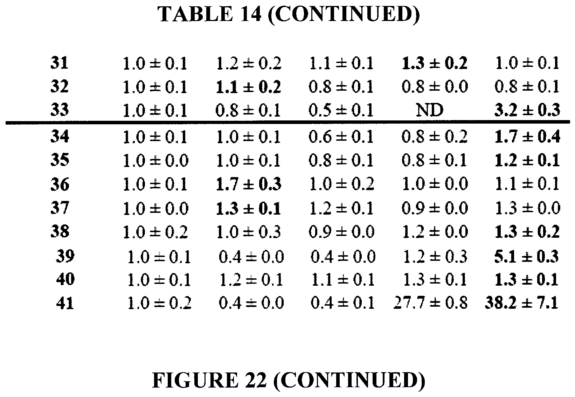

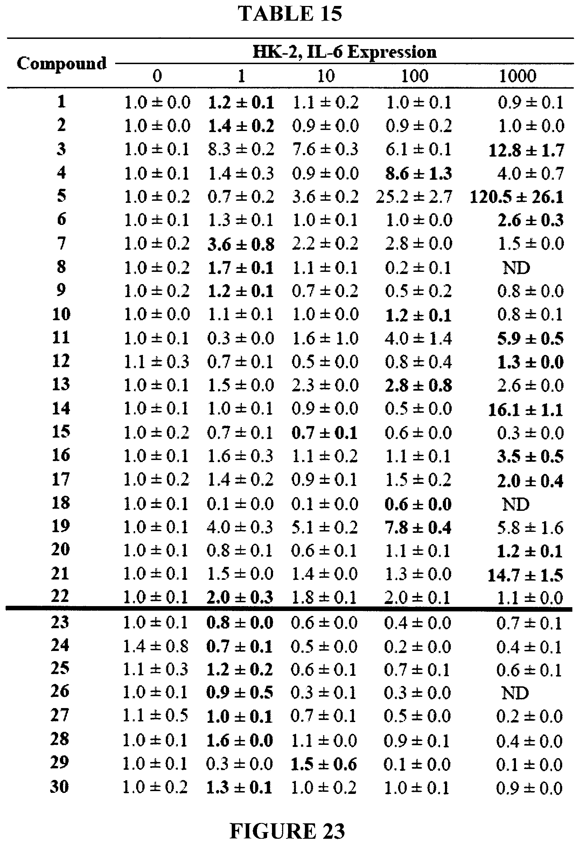

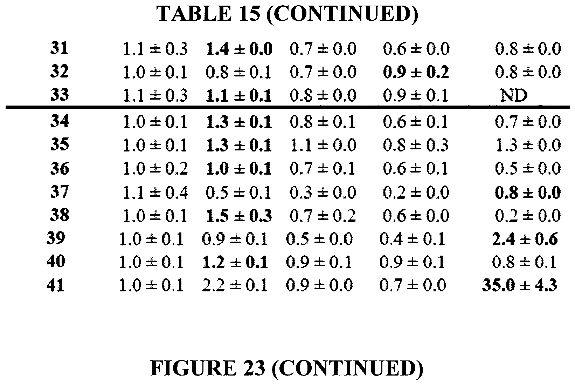

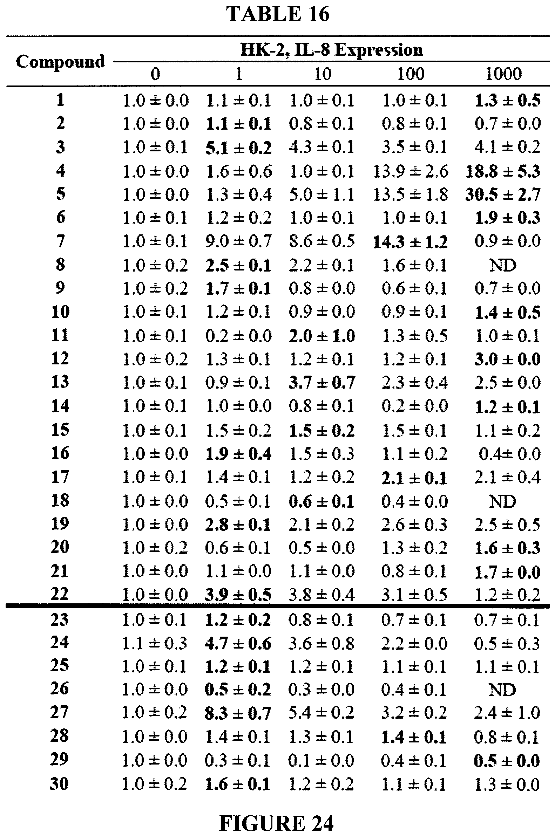

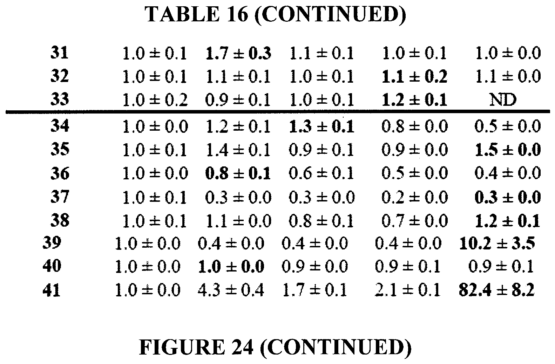

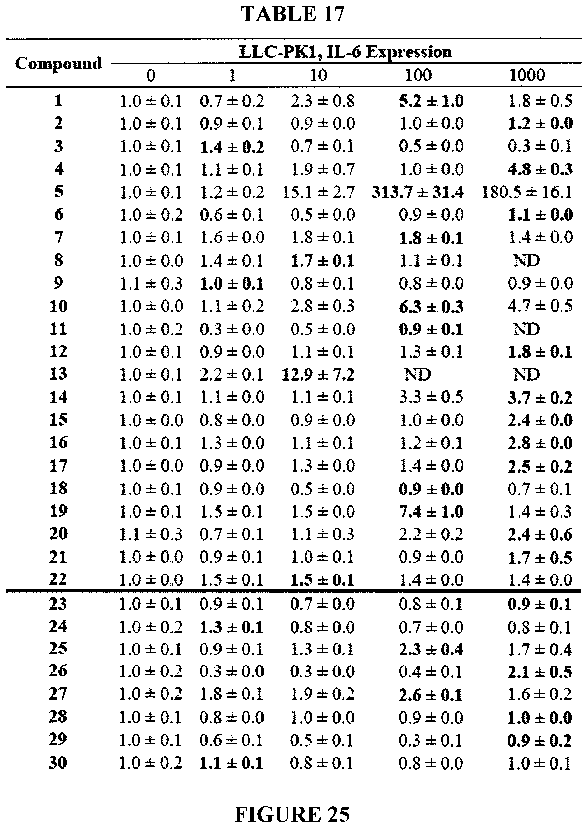

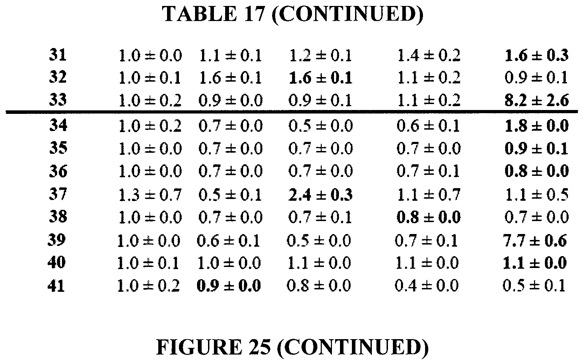

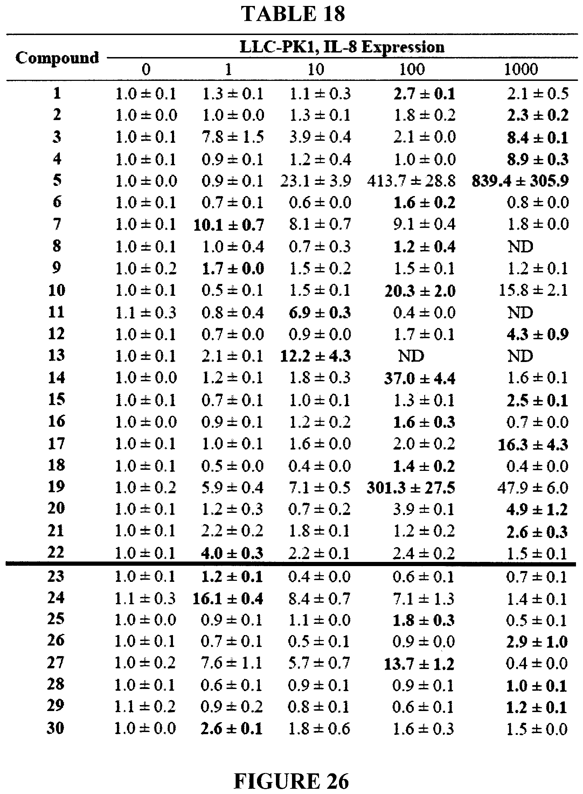

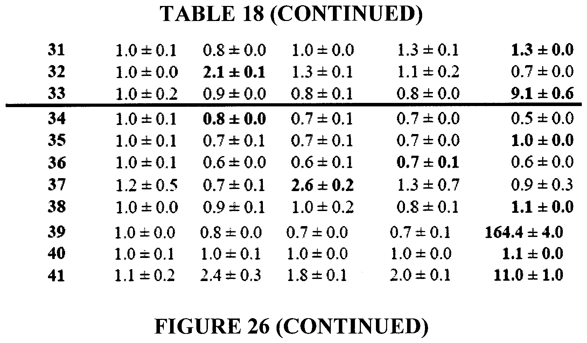

**Figures 17-26****. (Tables 9-18)** Expression levels of IL-6 and IL-8. Three different batches of HPTC (1-3) as well as HK-2 and LLC-PK1 cells were exposed to the 41 test compounds at concentrations of 1 µg/ml, 10 µg/ml, 100 µg/ml and 1000 µg/ml (vehicle control: 0 µg/ml drug concentration). The vehicle control contained the respective vehicle for the drug tested. The tables list the levels of IL-6 or IL-8 expression determined by qPCR. The numbers show the mean fold expression +/- s.d. (n = 3) relative to the vehicle control. In some cases the expression levels were not determined (ND) due to massive cell death. The highest levels of IL-6 or IL-8 expression that were determined for a given drug and cell type/batch combination when the whole range of drug concentrations was tested (1 µg - 1000 µg) are highlighted (bold). These highest expression values obtained with a specific drug and cell type/batch combination were entered into Tables 1 and 2.
**Figure 27****. (Table 19)** Details of primer pairs and amplicons. The sequences of the primer pairs (forward: F, reverse: R) for the different markers are shown. The sizes of the amplicons are provided in base pairs (bp).

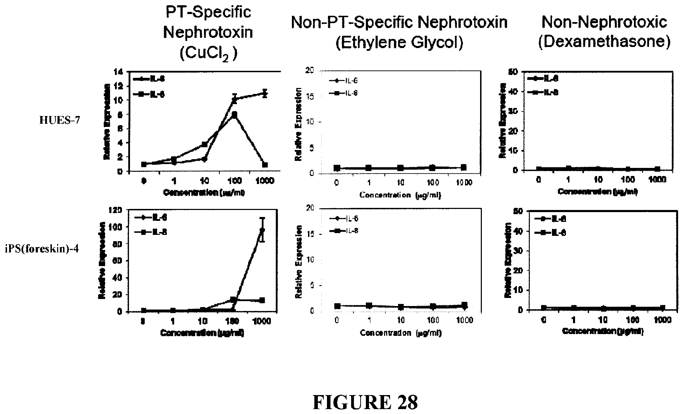

**Figure 28****.** Expression levels of IL-6 and IL-8 in HPTC-like cells derived from human embryonic stem cells (HUES-7) or human induced pluripotent stem cells (iPS(foreskin)-4). The test compounds CuCl₂ (PT-specific nephrotoxin), ethylene glycol (non-PT-specific nephrotoxin) and dexamethasone (not nephrotoxic) were applied at the concentrations indicated on the x-axis. Gene expression levels were determined by qPCR and are displayed as expression levels relative to the vehicle control (mean +/- s.d., n = 3, vehicle controls set to 1).

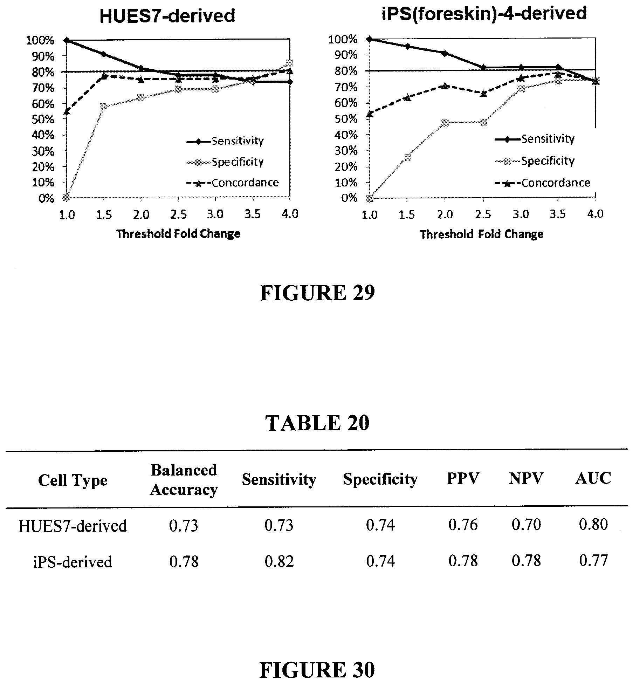

**Figure 29****.** Sensitivity, specificity and concordance with clinical data. HPTC-like cells derived from human embryonic stem cells (HUES-7) or human induced pluripotent stem cells (iPS(foreskin)-4) were tested with the set of 41 compounds listed in Table 1. Sensitivity, specificity and concordance with clinical data were determined as described with respect to HPTC (Figure 3 and Tables 3-5).
**Figure 30****. (Table 20)** Performance metrics obtained for HUES-7-derived or iPS(foreskin)-4-derived HPTC-like cells. The performance metrics were determined in the same way as described with respect to HPTC (Table 7).

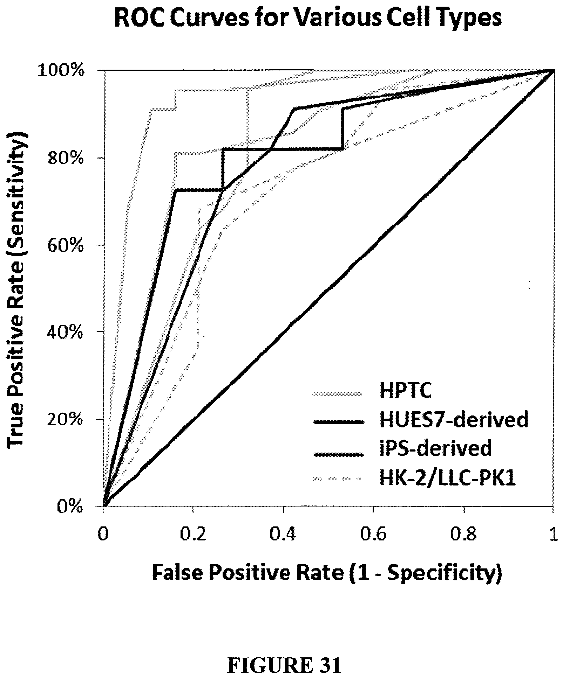

**Figure 31****.** ROC curves for each of the cell populations tested. The grey curves (HPTC and HK-2/LLC-PK1) are identical to those displayed in Figure 4F and are shown here for comparison.

### DETAILED DESCRIPTION

The assay methods described herein relate to use of renal proximal tubular cells *in vitro* to assess nephrotoxicity of compounds that damage the proximal tubule. The methods may be used to predict the toxicity of a compound for the renal proximal tubule *in vivo.*

The method uses expression levels of two cytokines, interleukin-6 (IL-6) and interleukin-8. Both IL-6 and IL-8 are expressed in the proximal tubule and in proximal tubular cells, both *in vivo* and *in vitro.* In the method, an increase in expression of one or both of IL-6 and IL-8 as compared to a control population indicates renal proximal tubular toxicity.

The method may provide sensitive and specific results, and may be performed using human primary renal proximal tubular cells (HPTCs). The method provides a model that thus may allow for predicting PT-specific toxicity in humans using IL-6 and/or IL-8 as an endpoint in combination with human renal proximal tubular cells (PTC). As well, PTC-like cells, derived from stem cells including human stem cells, may be used in the method.

Thus, there is provided a method of screening for nephrotoxicity of a compound, specifically for screening for renal proximal tubular (PT) toxicity. Briefly, the method comprises contacting a test population of renal proximal tubular cells and examining the test population of cells with a test compound that is to be assessed for PT toxcity. Subsequent to the contacting, expression levels of IL-6, IL-8, or both, in the test population are measured and compared to the expression levels in a control population of renal proximal tubular cells that has not been contacted with the test compound. If the expression levels of IL-6 or IL-8 or both are higher in the test population, the indication is that the test compound is a toxin that damages renal proximal tubular cells.

The cells used in the method, both for the test population and control population of cells, may be any type of PTC, or may be any type of PTC-like cells that have been differentiated from stem cells. The cells may be from any species that has renal proximal tubular cells, including a mammal, such as a pig or a human.

Thus, the cells may be derived from somatic cells. The cells may be from an established renal proximal tubular cell line, or may be primary renal proximal tubular cells. For example, the cells may be primary PTC isolated from kidney samples, including PTC isolated from a human kidney. The cells may be primary PTC obtained from a commercial source, including for example the American Type Culture Collection (ATCC). The cells may be an established renal proximal tubular cell line, including for example human kidney (HK)-2 cells or porcine LLC-PK1 cells.

In particular embodiments, the cells are human renal proximal tubular cells, either primary human renal proximal tubular cells (HPTCs) or from an established cell line.

Alternatively, the cells may be renal proximal tubular cell-like (PTC-like) cells differentiated from stem cells, including embryonic stem cells, adult stem cells such as mesenchymal stem cells, or induced pluripotent stem cells. PTC-like cells are cells that have been differentiated to express certain renal proximal tubular cell markers such as aquaporin (AQP)-1, which possess water transport functionality, CD13 (aminopeptidase N) and kidney-specific cadherin. PTC-like cells form differentiated and polarized epithelia sealed by tight junctions *in vitro* and generate tubular structures *in vitro* and *in vivo.* They display enzymatic functions typical for PTC and respond to parathyroid hormone. A method to differentiate stem cells into renal proximal tubular cell-like cells is published, for example in WO 2009/011663 and in Narayanan et al. (27). Commercially available stem cell lines may be used, including for example human embryonic stem cells.

In some instances, HPTC may be affected by inter-donor variability or may be difficult to obtain. Further, HPTC *in vitro* may often display a certain degree of dedifferentiation, which possibly reflects an injury-like state after cell isolation and could explain the lack of substantial up-regulation of novel AKI biomarkers after exposure to PT-specific nephrotoxins that may be observed. The use of stem-cell derived PTC-like cells in some embodiments may avoid some or all of the issues using HPTCs.

As used throughout this disclosure, reference to renal proximal tubular cells or PTC is intended to include reference to renal proximal tubular cell-like (PTC-like) cells that have been differentiated from stem cells, where context allows.

As will be appreciated, the same cell type should be used for the test population and control population.

As used herein, the term "cell" when referring to a renal proximal tubular cell or a renal proximal tubular-like cell is intended to refer to a single cell as well as a plurality or population of cells. Similarly, the term "cells" is also intended to refer to a single cell, where context allows.

Thus, in the method, cells are first cultured, in accordance with standard tissue culture methods. Methods for culturing are also described in the following Examples. Tissue culture conditions and techniques for renal proximal tubular cells are known. It should be noted that high serum concentrations in tissue culture medium may have an effect of causing the cells to de-differentiate. Thus, it may be advantageous to limit serum concentration in the tissue culture medium, for example to about 0.5% serum or less.

The test population of cells may be cultured in any format, including as a confluent monolayer, a subconfluent monolayer, a confluent epithelium, an organoid culture, a confluent 2D culture, an *in vitro* tubule, a 3D organoid culture, or a 3D culture including a static 3D culture or a 3D culture grown under microfluidic conditions.

In some embodiments, the cells are grown in a monolayer, such as a confluent or subconfluent monolayer. It should be noted that at cell densities of confluent monolayers, good cell differentiation can be achieved.

For example, cells may be seed at high density (e.g. 50 000 cells/cm²) in multi-well plates. The tissue culture polystyrene typically does not need any treating with a coating, gel etc. The cells may be cultivated for 3 days prior to contacting with the compound in order to provide the cells time to form a differentiated epithelium, which would be in the form of a confluent monolayer epithelium (as opposed to sub-confluent or multi-layered that may be used in other embodiments). Contacting with the test compound may then be performed overnight, for example for between about 8 hours and 16 hours, or even longer. An example of suitable culture conditions is provided for example in Li et al., Tox. Res, 2013 (DOI: 10.1039/c3tx50042j).

In some embodiments, the cells may be grown in microfluidic bioreactors, including in the form of a confluent monolayer or 2D confluent epithelium. A microfluidic bioreactor may be useful for long-term cultivation and repeated exposure to a test compound, as described herein. This format may be useful for generating compound concentration gradients within the culture.

In the method, the *in vitro* cultured cells are contacted with a compound that is to be tested for toxicity to PTC *in vivo.*

The test compound may be any compound that is to be assessed for PT-specific toxicity. The test compound may be any compound that is expected to come into contact with a subject, including being absorbed or ingested by a subject. For example, the test compound may be a pharmaceutical compound, an organic compound, a pesticide, an environmental toxin, a heavy metal-containing compound, an organic solvent, a food additive, a cosmetic or a nanoparticle.

The contacting may be done by adding the compound to the tissue culture medium in which the cells are cultured.

The contacting may be done over a period of time, for example by incubating the compound that is to be tested with the cells in culture. The contacting may be performed for about 8 hours or longer, for about 16 hours or longer, for 24 hours or longer, for 72 hours or longer.

The concentration of the test compound to be used may be varied, and may depend on the compound that is to be tested. Typically, when toxicity is observed *in vitro* in PTC at concentrations from about 1 µg/ml to about 1000 µg/ml, such toxicity tends to be predictive of PTC toxicity *in vivo* at clinically relevant concentrations. As set out in the Examples below, test compounds 1-22 display PT-specific toxicity in humans at clinically relevant concentrations.

For example, the test compound may be contacted with the population of cells at a concentration of about 0.001 µg/ml or higher, about 0.01 µg/ml or higher, about 0.1 µg/ml or higher, about 1 µg/ml or higher, about 10 µg/ml or higher, about 100 µg/ml or higher, or about 1000 µg/ml or higher. The test compound may be contacted with the population of cells at a concentration of from about 0.001 µg/ml to about 1000 µg/ml, from about 0.005 µg/ml to about 1000 µg/ml, or from about 0.01 µg/ml to about 500 µg/ml.

As will be appreciated, the control population of renal proximal tubular cells, although not contacted with the test compound, may be contacted with a negative control solution, for example the solvent or solution used to dissolve the test compound for contacting with the test population (vehicle control).

The contacting may be repeated. For example, the contacting may be performed two or more times, three or more times, four or more times or five or more times over a given period of time.

For example, after the first period of contacting is completed, the tissue culture medium may be replaced with fresh medium that contains the compound. Alternatively, the medium may be replaced with fresh medium that does not contain the test compound, and after a period of time with no contact, the test compound may then again be contacted with the test population of cells.

The contacting thus may be repeated one or more additional times (beyond the first instance of contacting), for example over a period of about 3 to about 14 days. The interval without any contact of test compound (i.e. exposing the cells to fresh medium) may last, for example, for one day to 14 days between the periods of contacting.

The contacting may be repeated shortly before or immediately before repeated prior to the determining of IL-6 and/or IL-8 expression levels.

After the contacting with the test compound, the test population is assessed with respect to expression levels of one or both of IL-6 and IL-8.

As will be appreciated, expression levels of IL-6 or IL-8 can be assessed by examining levels of expressed protein secreted by the cells, by examining levels of expressed mRNA within the cells, or by examining the expression levels of a reporter gene under control of the IL-6 or IL-8 regulatory regions.

Thus, in order to determine expression levels of the relevant interleukin, a sample of cells or culture medium from the test population is harvested, and protein levels or mRNA levels are detected for the relevant interleukin, or reporter protein levels expressed under control of the relevant interleukin are detected. As certain test compounds may affect protein synthesis, detection of mRNA levels may provide a more useful approach that may be applicable to a wider range of test compounds.

IL-6 and IL-8 are both secreted proteins and thus may be found in the cell culture medium in which the cells are cultured. Protein detection methods are known, and include for example ELISA techniques, biosensor techniques, electrochemical detection techniques, immunoblotting techniques, immunostaining techniques, cytometric bead array techniques, fluorescent label techniques and receptor binding techniques.

For example, a cell sample or culture medium sample containing secreted proteins may be collected and used in an immunoassay, with an α-IL-6 or α-IL-8 antibody used to capture or identify any IL-6 or IL-8 present in the sample. The immunoassay may include a detectable label, such as a fluorescent, coloured or radiolabelled marker either linked to the α-IL-6 or α-IL-8 antibody, or to a secondary antibody directed against the primary α-IL-6 or α-IL-8 antibody. The α-IL-6 or α-IL-8 antibody may be adsorbed on a surface, or the interleukin-antibody bound complex may be subsequently detected from the sample.

Sequences for IL-6 and IL-8 proteins for various different organisms are known. In some embodiments, the detected IL-6 protein comprises, consists or consists essentially of the sequence as set forth in SEQ ID NO: 1:

In some embodiments, the detected IL-6 protein comprises, consists or consists essentially of the sequence as set forth in SEQ ID NO: 2:

In some embodiments, the detected IL-6 protein comprises, consists or consists essentially of, a protein having about 75% or greater, about 80% or greater, about 85% or greater, about 90% or greater, about 95% or greater, about 96% or greater, about 97% or greater, about 98% or greater, or about 99% or greater sequence identity with SEQ ID NO: 1 or SEQ ID NO: 2, while still possessing the function of IL-6.

As used herein, "consists essentially of' or "consisting essentially of' means that the protein sequence includes one or more amino acid residues, including within the sequence or at one or both ends of the sequence, but that the additional amino acids do not materially affect the function of the protein.

In some embodiments, the detected IL-8 protein comprises, consists or consists essentially of the sequence as set forth in SEQ ID NO: 3:

In some embodiments, the detected IL-8 protein comprises, consists or consists essentially of the sequence as set forth in SEQ ID NO: 4:

In some embodiments, the detected IL-8 protein comprises, consists or consists essentially of, a protein having about 75% or greater, about 80% or greater, about 85% or greater, about 90% or greater, about 95% or greater, about 96% or greater, about 97% or greater, about 98% or greater, or about 99% or greater sequence identity with SEQ ID NO: 3 or SEQ ID NO: 4, while still possessing the function of IL-8.

RNA detection methods are also known, and include for example, quantitative PCR techniques, northern blot techniques, mircoarray techniques, TRAC techniques, fluorescent label techniques and phosphorimaging techniques.

For example, quantitative PCR (qPCR) techniques may be used to detect the levels of mRNA encoding IL-6 or IL-8 within a sample containing cells from the test population. Total RNA may be obtained from the cell sample, and cDNA may be generated by reverse transcription using the mRNA population within the cell as a template. The generated cDNA may then be used as a PCR template, using quantitative methods that allow for detection of a generated amplicon, for example by inclusion of fluorescent labels on the primers or on nucleotides that are incorporated into the generated amplicon.

Sequences for IL-6 and IL-8 mRNA for various different organisms are also known, and a skilled person will be able to detect the presence of an mRNA encoding IL-6 or IL-8.

In addition, IL-6 and/or IL-8 expression levels may be detected using molecular genetic techniques and transgenic PTC to detect levels of a reporter gene expressed under control of the IL-6 or IL-8 promoter/regulatory region. For example, reporter genes expressing a detectable product could be used that are under control of the IL-6 or IL-8 regulatory regions. Such reporter gene expression cassettes could be included in a vector such as a plasmid or viral vector, for transiently or stably transfecting or transducing a PTC population. Alternatively, knock-in expression cassettes could be made for inserting the reporter gene sequence into the genomic IL-6 and/or IL-8 loci of a PTC population. Molecular genetic techniques are known, and such techniques could be readily used in conjunction with the described *in vitro* methods, including with PTC-like cells differentiated from stem cells. Suitable reporter genes may include genes encoding green fluorescent protein (GFP) or luciferase, or other fluorescent proteins or proteins that are able to catalyse production of a detectable product. Sequences for IL-6 and IL-8 gene regulatory regions for various different organisms are known.

As can be seen from the above description, the use of computer-assisted detection techniques may assist in examining the IL-6 or IL-8 expression levels. The assay may be performed using robotic or automated devices or microfluidic devices in order to increase speed. IL-6 and IL-8 may be detected simultaneously for a given cell population, further increasing the speed and throughput of the assay. As well, if genetic methods are used to detect IL-6 or IL-8 expression levels, techniques such as high content screening may be used to visualize and measure expression levels of the reporter gene in the test population. High content screening techniques are known and used in the art. Such techniques may involve automated image analysis to assess levels of an expressed fluorescent protein under control of IL-6 and/or IL-8 regulation.

Once assessed, the IL-6 and/or IL-8 expression levels obtained for the test population is compared with the value obtained for the control population of renal proximal tubular cells under the same conditions minus the contact with the test compound. A change in expression levels of at least one of IL-6 and IL-8 in the test population relative to the control population is indicative that the test compound is a nephrotoxin that is directly toxic for renal proximal tubular cells. Thus, an increase in IL-6 or IL-8 expression in response to contact with the test compound can be seen to indicate that the test compound is a nephrotoxin that directly damages renal proximal tubular cells.

It may be desirable to set a threshold level for detection of the IL-6 or IL-8 expression levels. This may be done by using positive and negative control populations, as well as a set of compounds that are known to be non-nephrotoxic, a set of compounds that are known to be nephrotoxic but not specifically toxic to renal proximal tubular cells, and a set of compounds that are known to be specifically toxic to renal proximal tubular cells. The numbers of true positives, false positives, true negatives and false negatives can be determined as well as major performance metrics (PPV, NPV, sensitivity and specificity). The area under the curve values can be calculated and such ROC analysis methods are known and used in the art.

In addition, a threshold value may be determined in order to decide whether a test result is positive or negative. The threshold value relates to the fold increase in expression of IL-6 or IL-8 relative to the vehicle control.

For example, a test result may be positive if the increase in the expression level of at least one marker gene (IL-6 or IL-8) is similar to or greater than the threshold value. Optimal threshold values can be determined by testing broader ranges of threshold values, as shown in the Examples below, including in Figure 3. Actual threshold values may vary depending on the type of cell used and culture and contacting conditions used.

In some embodiments, the threshold (i.e the ratio of IL-6 expression or IL-8 expression levels in the test population to the same levels in the control population) is about 1.5 or greater, about 3.5 or greater, about 5 or greater, or about 10 or greater.

For any given test compound a dose response curve may be calculated by testing the compound at increasing concentrations and comparing the results for each concentration to results for a control population. In this way, an EC₅₀ or IC₅₀ value may be obtained for test compounds that are found to be toxic to renal proximal tubule cells.

The described methods may allow prediction of renal proximal tubule-specific toxicity in humans with high accuracy at an early pre-clinical stage, which has not previously been possible. Such prediction could provide additional valuable information during hit-to-lead discovery and lead optimization in developing pharmaceutical drugs, as well as allowing investigation of underlying mechanisms of PT-specific toxicity at an early stage. Pre-clinical results reliably predicting PT-specific toxicity would also help to design clinical studies and to decide whether a more extensive and more frequent clinical safety assessment would be required or patients with an increased risk of nephrotoxicity (e.g. advanced age, diabetes) should be excluded from Phase II studies until more information has been obtained. In this regard it is interesting to note that tetracycline consistently gave positive results when tested in the method, as described in the following examples. This drug has usually no obvious nephrotoxic effects, but can induce AKI and ESRD in patients with pre-existing kidney disease (51, 52). Novel biomarkers for detecting kidney toxicity (31-35) would be expected to be valuable in clinical studies where careful monitoring of nephrotoxic effects, as predicted by pre-clinical models, should be performed.

Thus, the methods described herein may be useful to predict compounds that will be toxic for PTC in humans at clinically relevant concentrations of the compound. As indicated above, compounds 1-10 and 19-22 described in the following example all displayed -PT-specific nephrotoxicity in humans at clinically relevant concentrations. The method identified most of these compounds as positive when used at concentrations of 1 µg/ml to 1000 µg/ml *in vitro.* Thus, at postitive test result when a compound is tested at concentrations ranging from 1 µg/ml to 1000 µg/ml *in vitro* may allow for prediction of PT-specific toxicity at clinically relevant concentrations *in vivo.*

The methods as described herein are further exemplified by way of the following non-limiting examples.

### EXAMPLES

### EXAMPLE 1

This model for the prediction of human renal proximal tubule toxicity employed HPTC and the expression levels of interleukin (IL)-6 and IL-8 were used as endpoint. The model was evaluated with 41 well-characterized drugs and chemicals. The results revealed that the predictability of this model is high and is in the range of about 76%-85%.

### Materials and Methods

**Test compounds:** 41 compounds were tested. The nature of these compounds, as well as their classification into different groups, is shown in Table 1 (Figure 9). Compounds 3-5, 8, 14, 18-20, 23, 30, 34 and 37 were obtained from Merck (Darmstadt, Germany). Compound 1 was purchased from PAA Laboratories GmbH (Pasching, Austria). Compound 10 was obtained from ChemService (West Chester, PA, USA) and compound 22 was purchased from Tocris Bioscience (Bristol, UK). All other test compounds were purchased from Sigma-Aldrich (St. Louis, MO, USA). Where possible stock solutions (10 mg/ml) of the test compounds were prepared with biotechnology grade water (1st Base, Singapore), which applied to the following compounds: 1,2, 4-6, 9-18, 23, 25, 28, 30-36, and 40. Otherwise, stock solutions (6.8 mg/ml -100 mg/ml depending on the solubility of the individual compound) were prepared with dimethyl sulfoxide (DMSO; Sigma-Aldrich; compounds 3, 7, 8, 19, 22, 27 and 41) or ethanol (compounds 20, 21, 24, 26, 29, 37 and 39). Vehicle controls were performed with the respective solvents. All stock solutions were stored in the dark at 4°C. Stock solutions of metal oxides and inorganic salts (compounds 11-16 and 18) were stored for up to 6 months. No stock solution of an organic compound was stored for longer than 3 months and most stock solutions were consumed much faster during the comprehensive test series.

**Cell culture:** HPTC were purchased from the American Type Culture Collection (ATCC, Manassas, VA, USA; HPTC 1) or were isolated from nephrectomy samples (HPTC 2-4) as described (26). Commercial HPTC (HPTC 1) were used at passage (P) 4 and P 5, and HPTC 2-4 were used at P 3 and P 4. Nephrectomy samples were derived from tumor patients and areas with normal tissue were selected for HPTC isolation after examination by a pathologist. Respective anonymized normal tissue samples were obtained from the Tissue Repository of the National University Health System (NUHS, Singapore). HK-2 and LLC-PK1 cells were purchased from ATCC. The different cell types were cultivated as described in the culture media recommended by the vendors (14). The culture medium used for HPTC contained 0.5% fetal bovine serum. Institutional Review Board approvals for the work with human kidney samples (DSRB-E/11/143) and the cell types (NUS-IRB Ref. Code: 09-148E) used have been obtained. All cells had been cryopreserved before use.

To ensure proper cell quality and marker expression patterns all batches of HPTC were assessed by quantitative real-time polymerase chain reaction (qPCR) using 31 marker genes (Figure 5). Expression of some markers was confirmed at the protein level by immunstaining and immunoblotting (Figure 6). For procedures, antibodies and primers see Table 19 (Figure 27) (and see also references 27, 28).

**qPCR:** Cells were seeded into 24-well microplates (Nalgene Nunc, Penfield, NY, USA) at a density of 50,000 cells/cm². Cells were cultivated for 72 h and were then treated for 16 h with the test compounds. Total RNA was isolated using NucleoSpin® RNA II (Macherey-Nagel, Düren, Germany) or RNeasy® Mini Kit (Qiagen, Hilden, Germany). cDNA synthesis was performed using SuperScript® III First Strand Synthesis System (Invitrogen, Carlsbad, CA, USA) and MyCycler® thermal cycler (Bio-Rad, Hercules, CA, USA). qPCR (up to 40 cycles) was then performed with the 7500 Fast Real-Time PCR System (Applied Biosystems, Carlsbad, CA, USA). Procedures were carried out according to the manufacturers' instructions with the software included in the device. The Sequence Detection Software 7500 Fast version 2.0.5 was used for data analysis. Gene expression levels were determined with the 2-ΔΔCT method. Primers were designed with the Primer Express Software version 3.0. Details of the primers used (purchased from Sigma-Aldrich) are provided in references 27, 28 and Table 19.

**High content screening (HCS):** HPTC and HK-2 cells were seeded into 96-well microplates (Becton Dickinson, Franklin Lakes, NJ, USA) at a density of 50,000 cells/cm² and LLC-PK1 cells were seeded at 16,000 cells/cm². Cells were cultivated for 72 h and were then treated for 16 h with the test compounds. After fixation for 10 min with 3.7% formaldehyde in phosphate-buffered saline, cell nuclei were stained with 4',6-diamidino-2-phenylindole (Merck) and imaged with the ImageXpress Micro High Content Screening System (Molecular Devices, Sunnyvale, CA, USA). 3 replicates were tested per cell type, drug and concentration. From each of the 3 wells 9 images were acquired. Cell nuclei were counted on each individual image and from these data the average cell numbers per well were derived. Data acquisition and analysis was performed by MetaXpress 2.0 (Molecular Devices, Sunnyvale, CA, USA).

**Data analysis:** Microsoft Office Excel 2003 was used for all calculations. Compounds were defined as positive in the *in vitro* model and predicted as PT-specific nephrotoxins if the increase of expression of at least one of the marker genes (IL-6 or IL-8) was equal to or higher than the threshold value at any of the compound concentrations tested. Threshold values between 0.3 and 4.0 were examined. Standard definitions are illustrated and provided in Figure 7. True positives (TP) were defined as PT-specific nephrotoxins in humans (Table 1, compounds 1-22, group 1) that gave positive results in the *in vitro* model. True negatives (TN) were defined as non-nephrotoxic compounds (Table 1, group 3, compounds 34-41) or nephrotoxic compounds that do not damage the PT in humans (Table 1, group 2, compounds 23- 33) that gave negative results in the *in vitro* model. The sensitivity was calculated by dividing the number of TP by the total number of PT-specific nephrotoxins (group 1, compounds 1-22). The specificity was calculated by dividing the number of TN by the total number of non-PT damaging compounds (groups 2 and 3, compounds 23-41). Balanced accuracy was defined as the mean of sensitivity and specificity. The positive predictive value (PPV) was calculated by dividing the number of TP by the total number of positives identified by the *in vitro* model. The negative predictive value (NPV) was calculated by dividing the number of TN by the total number of negatives identified by the *in vitro* model. The concordance with clinical data was calculated in the following way: TP + TN / total number of 41 drugs. When percentages were provided the numbers were multiplied with 100%. The receiver operating characteristic (ROC) curves were generated by plotting sensitivity against (1-specificity) at all threshold values ranging from 0.3 - 4.0 (same threshold values as in Figure 3). The unpaired t-test (Microsoft Office Excel 2010) was used for statistics. The normal distribution of the data was confirmed using SigmaStat (3.5) (Systat Software Inc., Chicago, IL, USA).

### Results

**Model design and endpoints:** We selected HPTC as the cellular model for method development to avoid issues related to animal cells and cell lines. All batches of HPTC were routinely characterized by microscopical examination and by qPCR, which was used to determine the expression levels of 31 different marker genes (see Figure 5 and Materials and Methods). For some markers, proper expression at the protein level was confirmed by immunostaining and immunoblotting (Figure 6). These analyses ensured a proper and comparable cell phenotype and quality. Cells were cultivated in normal uncoated multi-well plates. Uncoated tissue culture polystyrene sustains HPTC performance better than other materials with or without extracellular matrix coating (29, 30). Cells were seeded at high density and cultivated for three days before drug treatment to allow the formation of a differentiated epithelium, which was confirmed in control experiments (data not shown) as described before (14). The state of cell differentiation is of central importance for obtaining cell type-specific responses.

First, we determined suitable endpoints by assessing the expressional behavior of different marker genes related to PT injury. These included the mesenchymal marker vimentin (VIM). Kidney injury molecule-1 (KIM-1) and neutrophil gelatinase-associated lipocalin (NGAL) are both up-regulated in the tubular epithelium after injury and are potential novel biomarkers for the early detection of AKI (31-35). Interleukin (IL)-18 is up-regulated in the PT epithelium in diseased and injured kidneys and might be a useful biomarker for detecting kidney toxicity (31, 36, 37).

IL-6 and IL-8 are expressed in PT and PT-derived cells *in vivo* and *in vitro* (14, 38-41) and play a central role in pro-inflammatory processes, which occur after injury. Different studies demonstrated up-regulation of IL-6 and IL-8 in injured and diseased kidneys (42-44). It is thought that pro-inflammatroy cytokines play a central role in the pathophysiology of AKI, including nephrotoxin-induced AKI (45). Further, significant up-regulation of IL-6 after exposure to nephrotoxins has been demonstrate in a kidney culture model employing purified PTs (24).

To determine the effects of nephrotoxins on these six marker genes *in vitro* two different batches of HPTC were treated with high doses of gentamicin and CdCl₂ and expression levels were analyzed by qPCR. All individual results were normalized to the expression levels glyceraldehyde 3-phosphate dehydrogenase (GAPDH), which were consistent with cell numbers (Figure 8). If expression of a specific marker gene would be suitable as endpoint the gene should display relatively low expression levels in untreated cells and high levels of induction in response to nephrotoxins. In addition, the gene should be consistently up-regulated in different batches of HPTC and in response to different nephrotoxins. Figure 1 shows that these criteria were best fulfilled by IL-6 and IL-8. From the marker genes tested IL-8 displayed the highest levels of up-regulation after treatment with nephrotoxins.

Also NGAL showed consistent up-regulation, but the levels of up-regulation ranged only between 1.8-fold and 3.5-fold and were lower than the levels of up-regulation of IL-6 and IL-8. VIM was up-regulated in only one cell batch. KIM-1 and IL-18 were up-regulated in response to only one compound in one cell batch. The observed low level or lack of up-regulation of NGAL (Lipocalin-2) and KIM-1, respectively, was consistent with the results of other *in vitro* models employing human human renal proximal tubular cells and a PT-derived cell line (PREDICT-IV, 3rd and 4th Project Periodic Reports, June 30, 2012). It should be noted that primary cells, which are obtained by disruption of the organ, always display some degree of injury response. We observed that VIM, NGAL and KIM-1 were already expressed at relatively high levels in untreated control cells (Figure 5), which is consistent with previous results in case of VIM (46). This might explain the lack of or only moderate up-regulation after treatment with nephrotoxins. From all marker genes tested the expression levels of IL-6 and IL-8 were the lowest in untreated HPTC and were consistently < 0.1% of GAPDH expression.

**Predictive performance analyzed with 41 compounds:** Next, we determined the response to 41 well-characterized drugs and chemicals. Most of the 41 compounds used here were drugs that are routinely and widely applied in clinical practice. Some compounds, like CdCl₂ or lindane, are well-characterized environmental toxins and for all of the compounds a wealth of human and animal *in vivo* and *in vitro* data is available. As a starting point we selected compounds from published lists (2, 3, 18, 19) that classify compounds with regard to their nephrotoxicity in humans and their effects on various parts of the kidney and nephron. We then made an extensive literature search (PubMed) and also used Google and the ChemIDplus Advanced database to get further information on each selected compound and to confirm its classification.

22 compounds were classified as nephrotoxins that are known to directly damage the PT (group 1, Table 1, compounds 1-22; some of these drugs have also different negative effects on the kidney in addition to PT-specific injury). Further, the 41 compounds included 11 nephrotoxins that do not directly damage the PT and have other effects on the kidney (group 2, Table 1, compounds 23-33). In addition, 8 non-nephrotoxic drugs were included (group 3, Table 1, compounds 34-41). The assay was performed with three batches of HPTC derived from different donors and endpoints were expression of IL-6 and IL-8 determined by qPCR. For comparison, all experiments were also performed with HK-2 and LLC-PK1 cells (Table 1).

Drug exposure was performed for 16 hours after cultivating the cells for 3 days at confluent density. Initially, we tested a wide range of concentrations covering 5 orders of magnitude and ranging from 0.01 µg/ml to 1000 µg/ml. As usually no drug-induced changes (in comparison to the controls) were observed at the 2 lowest concentrations, we narrowed the range down and concentrations of 1 µg/ml, 10 µg/ml, 100 µg/ml and 1000 µg/ml were tested in all cases. Thus, the widest useful range of concentrations was applied in all experiments, with a lack of drug-induced changes at concentrations below the lower limit and compromised solubility of many compounds at concentrations exceeding the upper limit. All results were normalized to the vehicle control and expressed as fold change of IL-6 and IL-8 expression.

Dose-response curves obtained with HPTC and three drugs selected from each group are shown in Figure 2. Detailed results on IL-6 and IL-8 expression levels for each cell batch/type and each drug at every concentration tested are listed in Tables 9-18 (Figures 17-26). The highest levels of IL-6 and IL-8 expression determined for each drug and cell batch/type at any given concentration of a drug within the range tested are highlighted in Tables 9-18. These highest expression levels are summarized in Tables 1 and 2. The results showed that different drugs had different effects on the expression of IL-6 and IL-8, and some drugs induced both marker genes, while other drugs induced only one or none of the marker genes (Figure 2). Expression of at least one marker gene was often substantially increased after exposure to PT-specific nephrotoxins (group 1, Tables 1 and 2 (Figures 9 and 10), Figure 2), whereas gene expression levels typically remained low at all concentrations after exposure to drugs from groups 2 and 3 (Tables 1 and 2, Figure 2). Overall comparable results were obtained when secretion of IL-6 and IL-8 was assessed by ELISA (data not shown). However, many of the drugs used inhibited protein synthesis and therefore qPCR data were more reliable.

In order to classify a result obtained with a specific drug and cell type/batch as positive or negative it was determined whether the highest expression level (mean; Tables 1 and 2) was equal or higher than a threshold value. A drug was classified as positive and predicted as PT-specific nephrotoxin if the highest increase in gene expression (Tables 1 and 2) of at least one of the markers (IL-6 and IL-8) was equal or higher than the threshold value. As it was unclear which threshold value might be most appropriate this analysis was performed for a range of threshold levels from 0.3 to 4.0.

Examples that illustrate the processing of the data are shown in Tables 3 and 4. Both tables display the same data set obtained with HPTC 1. This data set is identical with the HPTC 1 data set in Table 2 and shows the highest expression levels of IL-6 and IL-8. A threshold of 2.0 (Table 3 (Figure 11)) or 3.5 (Table 4 (Figure 12)), respectively, was applied to this data set. If the expression level of at least one of the marker genes was equal to or higher than the threshold the test result was classified as positive, and the classification is indicated in Tables 3 and 4. Based on these results the sensitivity (number of positive test results from group 1 compounds (TP) / total number of 22 group 1 compounds) and the specificity (number of negative test results from group 2 and 3 compounds (TN) / total number of 19 group 2 and 3 compounds) were calculated.

In this way, the sensitivity and specificity were calculated for every cell type and batch at 7 different threshold levels. The results are summarized in Table 5 (Figure 13) and are graphically displayed in Figure 3. In addition, Figure 3 shows the overall concordance with clinical data.

The results revealed that a threshold value of 3.5 was most suitable for two of the HPTC batches (Figure 3; HPTC 1 and 2). At this threshold level sensitivity, specificity and overall concordance with clinical data were ∼ 90% (HPTC 1) and ∼ 80% (HPTC 2). If the same threshold value (3.5) was applied to the third batch (HPTC 3) the specificity was still ∼ 80%, whereas sensitivity and overall concordance with clinical data were ∼ 64% and ∼ 71%, respectively. As expected, these data revealed some inter-donor variability between the different batches of primary cells. Compared to HPTC the optimal threshold levels for HK-2 and LLC-PK1 cells were different and overall the data revealed that the predictability was lower when these cell lines were used instead of HPTC (Figure 3). For instance, at threshold values of 3 and above, which were most suitable for LLC-PK1 cells, all of the values remained below 80% and ranged between ∼ 64% and ∼ 74%.

To further analyze the predictability we calculated the ROC curves and determined the area under curve (AUC) values. Figure 4 shows the ROC curves obtained with each cell batch/line and the results for either IL-6 or IL-8 or the combination of markers are displayed. The respective AUC values are shown in Table 6 (Figure 14). The results confirm that the predictability was higher when HPTC were used (compared to HK-2 and LLC-PK1 cells), and this applied to the mean and median values as well as to each single batch of HPTC. Use of a combination of both markers only slightly improved the results in comparison to the use of IL-8 alone. The AUC values obtained with the marker combination ranged from 0.71 (HK-2) to 0.94 (HPTC 1).

The most important performance metrics (balanced accuracy, sensitivity, specificity, PPV, NPV and AUC values) are summarized in Table 7 (Figure 15). For Table 7 and further analyses we used the combined expression data of IL-6 and IL-8 as endpoint. The mean PPV for HPTC was 0.85, which means that 85% of the time a compound was called out as PT-specific toxin correctly. The respective values for HK-2 and LLC-PK1 cells were 0.73 and 0.74, respectively. The mean NPV of HPTC was 0.79. Here, the values for the cell lines were 0.6 (HK-2) and 0.67 (LLC-PK1).

**Impact of endpoints:** Next, we compared assay performance when either IL-6/IL-8 expression or cell death were used as endpoint. Cell numbers were determined by high content screening (HCS) in order to quantify cell death. Table 8 (Figure 16) summarizes the results. Using IL-6/IL-8 expression as endpoint 91% (20/22) of the PT-specific nephrotoxins gave a positive result and were correctly predicted when HPTC 1 were used. In contrast, substantial cell death, which allowed to calculate IC50 values, was observed in only 42% (8/19) of cases tested. Even when all of these cases, where > 50% cell death occurred, would be classified as positives, the sensitivity would not be better than chance. The same applied to the results obtained with HK-2 and LLC-PK1 cells, where with respect to group 1 compounds substantial cell death was observed in 43% (6/14) and 53% (8/15) of cases tested.

These results support the idea that endpoints measuring general cytotoxicity might not be useful for organ-specific assays. It should be noted that cell death, as measured here by determining cell numbers, is also measured by other widely used assays such as the neutral red uptake assay or the MTT assay (the MTT assay measures metabolic activity, which is often used as an indirect indicator of cell numbers). In the light of these results we refrained from addressing other endpoints measuring general cytotoxicity.

Here, we developed a model for the prediction of PT-specific toxicity in humans. The model was based on HPTC and tested with 41 compounds. For comparison, HK-2 and LLC-PK1 cells were also assessed. When three batches of HPTC were used the mean and median values for the major performance metrics (balanced accuracy, sensitivity, specificity, PPV, NPV and AUC values) ranged between 0.76 and 0.85. These results show that the predictability of the model is high and it would be expected that in ∼ 76% - 85% of cases where compounds were predicted as positives or negatives the predictions would be correct.

Two major features distinguish our model from other models for the prediction of PT-specific toxicity: i) the use of HPTC and ii) use of IL-6/IL-8 expression as endpoint. Another difference is the application of confluent epithelia, but the impact of this parameter is currently unclear. Our results show that both, the use of HPTC and of the selected endpoint provided better results. The key performance metrics only ranged between 0.60 and 0.79 when HK-2 or LLC-PK1 cells were applied instead of HPTC. Also, the sensitivity was severely compromised when cell death was used as endpoint instead of IL-6/IL-8 expression. IL-6 and IL-8 are expressed by a large variety of cell types in response to a broad variety of stimuli and injury mechanisms (see, for instance 47-50 and citations therein) and thus the specificity of our model might be surprising. However, potential contributions from other cell types do not play a role in an *in vitro* model based on one cell type. As long as no other parameters than HPTC injury increase marker gene expression in the *in vitro* model the response would be expected to be specific.

### EXAMPLE 2

This experiment was performed using two types of renal proximal tubular-like cells, under conditions as described in Example 1 above, using the same identified 41 compounds. The two cell types were cell populations differentiated from human embryonic stem cells (HUES-7 cells available from Howard Hughes Medical Institute) and from human induced pluripotent cells, which were derived from human foreskin (iPS(foreskin)-4; WiCell Research Institute, Wisconsin, USA). Both stem cell types were differentiated into renal proximal tubular-like cells using a previously described method (see reference 27).

Results for selected compounds tested on both types of cell populations are shown in Figure 28.

At threshold = 3.5, both cell populations of renal proximal tubular-like cells showed > 70% sensitivity and specificity and overall concordance with human clinical data, as seen in Figures 29 and 30. The major performance metrics of the assay using stem cell-derived renal proximal tubular-like cells ranged between 0.70 and 0.82 (Figure 30). ROC curves for each of the cell populations tested are shown in Figure 31. Further improvements of the assay are expected by optimizing the protocol for the differentiation of stem cells in renal proximal tubular-like cells.

As used in this specification and the appended claims, the singular forms "a", "an" and "the" include plural reference unless the context clearly dictates otherwise. As used in this specification and the appended claims, the terms "comprise", "comprising", "comprises" and other forms of these terms are intended in the non-limiting inclusive sense, that is, to include particular recited elements or components without excluding any other element or component. As used in this specification and the appended claims, all ranges or lists as given are intended to convey any intermediate value or range or any sublist contained therein. Unless defined otherwise all technical and scientific terms used herein have the same meaning as commonly understood to one of ordinary skill in the art to which this invention belongs.

### References

1. E. M. Levy, C. M. Viscoli and R. I. Horwitz, JAMA, 1996, 275, 1489-1494.
2. D. Choudhury and Z. Ahmed, Nat Clin Pract Nephrol, 2006, 2, 80-91.
3. X. Guo and C. Nzerue, Cleve Clin J Med, 2002, 69, 289-290, 293-284, 296-287 passim.
4. K. Nash, A. Hafeez and S. Hou, Am J Kidney Dis, 2002, 39, 930-936.
5. B. S. Moffett and S. L. Goldstein, Clin JAm Soc Nephrol, 2011, 6, 856-863.
6. C. C. Szeto and K. M. Chow, Ren Fail, 2005, 27, 329-333.
7. M. A. Perazella, Hosp Pract (Minneap), 2001, 36, 43-46, 55-46.
8. W. S. Redfern, L. Ewart, T. G. Hammond, R. Bialecki, L. Kinter, S. Lindgren, C. E. Pollard, R. Roberts, M. G. Rolf and J. P. Valentin, The Toxicologist, 2010, 114, 231.
9. H. Izzedine, M. Harris and M. A. Perazella, Nat Rev Nephrol, 2009, 5, 563-573.
10. P. P. Kapitsinou and N. Ansari, J Med Case Rep, 2008, 2, 94.
11. W. Pfaller and G. Gstraunthaler, Environ Health Perspect, 1998, 106 Suppl 2, 559-569.
12. P. Prieto, Altern Lab Anim, 2002, 30 Suppl 2, 101-106.
13. Y. Wu, D. Connors, L. Barber, S. Jayachandra, U. M. Hanumegowda and S. P. Adams, Toxicol In Vitro, 2009, 23, 1170-1178.
14. Y. Li, Y. Zheng, K. Zhang, J. Y. Ying and D. Zink, Nanotoxicology, 2012, 6, 121-133.
15. P. H. Bach, D. K. Obatomi and S. Brant, In vitro methods for nephrotoxicity screening and risk assessment, Academic Press Ltd, San Diego, 1997.
16. M. Bens and A. Vandewalle, Pflugers Arch, 2008, 457, 1-15.
17. S. E. Jenkinson, G. W. Chung, E. van Loon, N. S. Bakar, A. M. Dalzell and C. D. Brown, Pflugers Arch, 2012, 464, 601-611.
18. Z. Lin and Y. Will, Toxicol Sci, 2012, 126, 114-127.
19. T. Duff, S. Carter, G. Feldman, G. McEwan, W. Pfaller, P. Rhodes, M. Ryan and G. Hawksworth, Altern Lab Anim, 2002, 30 Suppl 2, 53-59.
20. W. Li, D. F. Choy, M. S. Lam, T. Morgan, M. E. Sullivan and J. M. Post, Toxicol In Vitro, 2003, 17, 107-113.
21. W. Li, M. Lam, D. Choy, A. Birkeland, M. E. Sullivan and J. M. Post, Toxicol In Vitro, 2006, 20, 669-676.
22. Limonciel, L. Aschauer, A. Wilmes, S. Prajczer, M. O. Leonard, W. Pfaller and P. Jennings, Toxicol In Vitro, 2011, 25, 1855-1862.
23.1. Astashkina, B. K. Mann, G. D. Prestwich and D. W. Grainger, Biomaterials, 2012, 33, 4712-4721.
24.1. Astashkina, B. K. Mann, G. D. Prestwich and D. W. Grainger, Biomaterials, 2012, 33, 4700-4711.
25. C. Beeson, G. C. Beeson and R. G. Schnellmann, Anal Biochem, 2010, 404, 75-81.
26. D. A. Vesey, W. Qi, X. Chen, C. A. Pollock and D. W. Johnson, Methods Mol Biol, 2009, 466, 19-24.
27. K. Narayanan, K. M. Schumacher, F. Tasnim, K. Kandasamy, A. Schumacher, M. Ni, S. Gao, B. Gopalan, D. Zink and J. Y. Ying, Kidney Int, 2013, 83, 593-603.
28. F. Tasnim, K. Kandasamy, J. S. Muck, M. S. Bin Ibrahim, J. Y. Ying and D. Zink, Tissue Eng Part A, 2012, 18, 262-276.
29. M. Ni, J. C. Teo, M. S. Ibrahim, K. Zhang, F. Tasnim, P. Y. Chow, D. Zink and J. Y. Ying, Biomaterials, 2011, 32, 1465-1476.
30. M. Ni, P. K. Zimmermann, K. Kandasamy, W. Lai, Y. Li, M. F. Leong, A. C. Wan and D. Zink, Biomaterials, 2012, 33, 353-364.
31. J. V. Bonventre, V. S. Vaidya, R. Schmouder, P. Feig and F. Dieterle, Nat Biotechnol, 2010, 28, 436-440.
32. F. Dieterle, F. Sistare, F. Goodsaid, M. Papaluca, J. S. Ozer, C. P. Webb, W. Baer, A. Senagore, M. J. Schipper, J. Vonderscher, S. Sultana, D. L. Gerhold, J. A. Phillips, G. Maurer, K. Carl, D. Laurie, E. Harpur, M. Sonee, D. Ennulat, D. Holder, D. Andrews-Cleavenger, Y. Z. Gu, K. L. Thompson, P. L. Goering, J. M. Vidal, E. Abadie, R. Maciulaitis, D. Jacobson-Kram, A. F. Defelice, E. A. Hausner, M. Blank, A. Thompson, P. Harlow, D. Throckmorton, S. Xiao, N. Xu, W. Taylor, S. Vamvakas, B. Flamion, B. S. Lima, P. Kasper, M. Pasanen, K. Prasad, S. Troth, D. Bounous, D. Robinson-Gravatt, G. Betton, M. A. Davis, J. Akunda, J. E. McDuffie, L. Suter, L. Obert, M. Guffroy, M. Pinches, S. Jayadev, E. A. Blomme, S. A. Beushausen, V. G. Barlow, N. Collins, J. Waring, D. Honor, S. Snook, J. Lee, P. Rossi, E. Walker and W. Mattes, Nat Biotechnol, 2010, 28, 455-462.
33. T. Ichimura, C. C. Hung, S. A. Yang, J. L. Stevens and J. V. Bonventre, Am J Physiol Renal Physiol, 2004, 286, F552-563.
34. W. S. Waring and A. Moonie, Clin Toxicol (Phila), 2011, 49, 720-728.
35. J. Mishra, Q. Ma, A. Prada, M. Mitsnefes, K. Zahedi, J. Yang, J. Barasch and P. Devarajan, JAm Soc Nephrol, 2003, 14, 2534-2543.
36. D. Liang, H. F. Liu, C. W. Yao, H. Y. Liu, C. M. Huang-Fu, X. W. Chen and S. H. Du, Nephrology (Carlton), 2007, 12, 53-61.
37. K. Miyauchi, Y. Takiyama, J. Honjyo, M. Tateno and M. Haneda, Diabetes Res Clin Pract, 2009, 83, 190-199.
38. Z. I. Niemir, H. Stein, A. Ciechanowicz, P. Olejniczak, G. Dworacki, E. Ritz, R. Waldherr and S. Czekalski, Am J Kidney Dis, 2004, 43, 983-998.
39. J. S. Gerritsma, P. S. Hiemstra, A. F. Gerritsen, W. Prodjosudjadi, C. L. Verweij, L. A. Van Es and M. R. Daha, Clin Exp Immunol, 1996, 103, 289-294.
40. J. S. Gerritsma, C. van Kooten, A. F. Gerritsen, A. M. Mommaas, L. A. van Es and M. R. Daha, Exp Nephrol, 1998, 6, 208-216.
41. S. C. Tang, J. C. Leung and K. N. Lai, Contrib Nephrol, 2011, 170, 124-134.
42. M. Araki, N. Fahmy, L. Zhou, H. Kumon, V. Krishnamurthi, D. Goldfarb, C. Modlin, S. Flechner, A. C. Novick and R. L. Fairchild, Transplantation, 2006, 81, 783-788.
43. D. N. Grigoryev, M. Liu, H. T. Hassoun, C. Cheadle, K. C. Barnes and H. Rabb, J Am Soc Nephrol, 2008, 19, 547-558.
44. D. Tramma, M. Hatzistylianou, G. Gerasimou and V. Lafazanis, Pediatr Nephrol, 2012, 27, 1525-1530.
45. A. Akcay, Q. Nguyen and C. L. Edelstein, Mediators Inflamm, 2009, 2009, 137072.
46. G. Elberg, S. Guruswamy, C. J. Logan, L. Chen and M. A. Turman, Cell Tissue Res, 2008, 331, 495-508.
47. T. A. Luger and T. Schwarz, J Invest Dermatol, 1990, 95, 100S-104S.
48. P. Trayhurn, C. A. Drevon and J. Eckel, Arch Physiol Biochem, 2011, 117, 47-56.
49. M. Rossol, H. Heine, U. Meusch, D. Quandt, C. Klein, M. J. Sweet and S. Hauschildt, Crit Rev Immunol, 2011, 31, 379-446.
50. R. A. Swerlick and T. J. Lawley, J InvestDermatol, 1993, 100, 111S-115S.
51. C. S. Miller and G. J. McGarity, J Am Dent Assoc, 2009, 140, 56-60.
52. M. E. Phillips, J. B. Eastwood, J. R. Curtis, P. C. Gower and H. E. De Wardener, Br Med J, 1974, 2, 149-151.
53. A. Saito, K. Sawada and S. Fujimura, Hemodial Int, 2011, 15, 183-192.

## Claims

1. An *in vitro* method for screening renal proximal tubular cell specific toxicity of a compound, the method comprising:
contacting a test compound with a test population of renal proximal tubular cells, preferably for a period of time of about 8 hours or longer, the test population being a confluent monolayer, a subconfluent monolayer, a confluent epithelium or a 2D confluent culture; and
determining the expression level of an interleukin in the test population, the interleukin being (i) interleukin-6 (IL-6); or (ii) interleukin-8 (IL-8); or both (i) and (ii);
wherein expression levels of the interleukin in the test population being greater than expression levels in a control population of renal proximal tubular cells not contacted with the test compound is indicative that the test compound is toxic for renal proximal tubular cells.

2. The method of claim 1, wherein said determining of said expression level of the interleukin comprises determining the levels of mRNA encoding the interleukin.

3. The method of claim 2, wherein said determining comprises one or more of quantitative PCR techniques, northern blot techniques, microarray techniques, TRAC techniques, fluorescent label techniques and phosphorimaging techniques.

4. The method of claim 1, wherein said determining of said expression level of the interleukin comprises determining the level of secreted interleukin protein.

5. The method of claim 4, wherein said determining comprises one or more of ELISA techniques, biosensor techniques, electrochemical detection techniques, immunoblotting techniques, cytometric bead array techniques, fluorescent label techniques, and receptor binding techniques.

6. The method of claim 1, wherein said determining of said expression level of the interleukin comprises detecting levels of a reporter gene expressed under control of IL-6 or IL-8 regulation.

7. The method of any one of claims 1 to 6, wherein the ratio of expression levels of the interleukin in the test population to expression levels of the interleukin in the control population being about 1.5 or greater, preferably 3.5 or greater, is indicative that the test compound is toxic for renal proximal tubular cells.

8. The method of any one of claims 1 to 7, wherein the renal proximal tubular cells are derived from somatic cells and are primary cells or are cells from a stable cell line.

9. The method of claim 8, wherein the renal proximal tubular cells are human primary renal proximal tubular cells, HK-2 cells, or LLC-PK1 cells.

10. The method of any one of claims 1 to 7, wherein the renal proximal tubular cells are derived from stem cells and are differentiated from embryonic stem cells, mesenchymal stem cells, or induced pluriopotent stem cells.

11. The method of any one of claims 1 to 10, wherein the proximal tubular cells are human renal proximal tubular cells.

12. The method of any one of claims 1 to 10, wherein the proximal tubular cells are non-human proximal tubular cells.

13. The method of any one of claims 1 to 12, wherein said contacting is repeated one or more times in a period of from about 3 to about 14 days.

14. The method of any one of claims 1 to 13, wherein said contacting comprises adding the test compound to the test population of renal proximal tubular cells at a concentration of about 0.001 to about 1000 µg/ml.

## Patentansprüche

1. *In-vitro*-Verfahren zum Screening auf spezifische Toxizität für renale proximale tubuläre Zellen einer Verbindung, wobei das Verfahren umfasst:
Inkontaktbringen einer Testverbindung mit einer Testpopulation von renalen proximalen tubulären Zellen, vorzugsweise für einen Zeitraum von ungefähr 8 Stunden oder länger, wobei die Testpopulation eine konfluente Monoschicht, eine subkonfluente Monoschicht, ein konfluentes Epithel oder eine 2D konfluente Kultur ist; und
Bestimmen des Expressionsniveaus eines Interleukins in der Testpopulation, wobei das Interleukin (i) lnterleukin-6 (IL-6); oder (ii) Interleukin-8 (IL-8); oder sowohl (i) als auch (ii) ist;
wobei die Expressionsniveaus des Interleukins in der Testpopulation, die größer als die Expressionsniveaus in einer Kontrollpopulation von renalen proximalen tubulären Zellen sind, die nicht mit der Testverbindung in Kontakt gebracht wurden, darauf hinweisen, dass die Testverbindung für renale proximale tubuläre Zellen toxisch ist.

2. Verfahren nach Anspruch 1, wobei die Bestimmung des Expressionsniveaus des Interleukins die Bestimmung der für das Interleukin kodierenden mRNA-Spiegel umfasst.

3. Verfahren nach Anspruch 2, wobei die Bestimmung eine oder mehrere quantitative PCR-Techniken, Northern Blot-Techniken, Microarray-Techniken, TRAC-Techniken, Fluoreszenzmarkierungstechniken und Phosphorimaging-Techniken umfasst.

4. Verfahren nach Anspruch 1, wobei die Bestimmung des Expressionsniveaus des Interleukins die Bestimmung des Spiegels des sekretierten Interleukin-Proteins umfasst.

5. Verfahren nach Anspruch 4, wobei die Bestimmung eine oder mehrere ELISA-Techniken, Biosensor-Techniken, elektrochemische Nachweistechniken, Immunblotting-Techniken, zytometrische Bead-Array-Techniken, Fluoreszenzmarkierungstechniken und Rezeptorbindungstechniken umfasst.

6. Verfahren nach Anspruch 1, wobei die Bestimmung des Expressionsniveaus des Interleukins den Nachweis von Niveaus eines Reportergens umfasst, das unter der Kontrolle der IL-6- oder IL-8-Regulierung exprimiert wird.

7. Verfahren nach einem der Ansprüche 1 bis 6, wobei das Verhältnis der Expressionsniveaus des Interleukins in der Testpopulation zu den Expressionsniveaus des Interleukins in der Kontrollpopulation ungefähr 1,5 oder höher, vorzugsweise 3,5 oder höher ist, was anzeigt, dass die Testverbindung für renale proximale tubuläre Zellen toxisch ist.

8. Verfahren nach einem der Ansprüche 1 bis 7, wobei die renalen proximalen tubulären Zellen aus somatischen Zellen abgeleitet sind und primäre Zellen sind oder Zellen aus einer stabilen Zelllinie sind.

9. Verfahren nach Anspruch 8, wobei die renalen proximalen tubulären Zellen menschliche primäre renale proximale tubuläre Zellen, HK-2-Zellen oder LLC-PK1-Zellen sind.

10. Verfahren nach einem der Ansprüche 1 bis 7, wobei die renalen proximalen tubulären Zellen aus Stammzellen abgeleitet sind und sich aus embryonalen Stammzellen, mesenchymalen Stammzellen oder induzierten pluripotenten Stammzellen differenziert haben.

11. Verfahren nach einem der Ansprüche 1 bis 10, wobei die proximalen tubulären Zellen menschliche renale proximale tubuläre Zellen sind.

12. Verfahren nach einem der Ansprüche 1 bis 10, wobei die proximalen tubulären Zellen nicht-menschliche proximale tubuläre Zellen sind.

13. Verfahren nach einem der Ansprüche 1 bis 12, wobei das Inkontaktbringen ein oder mehrmals in einem Zeitraum von ungefähr 3 bis ungefähr 14 Tagen wiederholt wird.

14. Verfahren nach einem der Ansprüche 1 bis 13, wobei das Inkontaktbringen die Zugabe der Testverbindung zu der Testpopulation der renalen proximalen tubulären Zellen in einer Konzentration von ungefähr 0,001 bis ungefähr 1.000 µg/mL umfasst.

## Revendications

1. Procédé in vitro de dépistage de la toxicité spécifique d'un composé envers les cellules tubulaires proximales rénales, le procédé comprenant:
la mise en contact d'un composé d'essai avec une population d'essai de cellules tubulaires proximales rénales, préférablement pendant une durée d'environ 8 heures ou plus, la population d'essai étant une monocouche confluente, une monocouche subconfluente, un épithélium confluent ou une culture confluente 2D; et
la détermination du niveau d'expression d'une interleukine dans la population d'essai, l'interleukine étant (i) l'interleukine-6 (IL-6); ou (ii) l'interleukine-8 (IL-8); ou à la fois (i) et (ii);
où des niveaux d'expression de l'interleukine dans la population d'essai qui sont supérieurs aux niveaux d'expression dans une population témoin de cellules tubulaires proximales rénales non mise en contact avec le composé d'essai indiquent que le composé d'essai est toxique pour les cellules tubulaires proximales rénales.

2. Procédé selon la revendication 1, où ladite détermination dudit niveau d'expression de l'interleukine comprend la détermination des niveaux d'ARNm codant pour l'interleukine.

3. Procédé selon la revendication 2, où ladite détermination comprend l'une ou plusieurs parmi les techniques ACP quantitative, les techniques Northern blot, les techniques des biopuces, les techniques TRAC, les techniques des marqueurs fluorescents et les techniques d'imagerie au phosphore.

4. Procédé selon la revendication 1, où ladite détermination dudit niveau d'expression de l'interleukine comprend la détermination du niveau de protéine d'interleukine secrétée.

5. Procédé selon la revendication 4, où ladite détermination comprend l'une ou plusieurs parmi les techniques ELISA, les techniques des biocapteurs, les techniques de détection électrochimiques, les techniques d'immunoempreinte, les techniques CBA (Cytometric Bead Array), les techniques des marqueurs fluorescents et les techniques de fixation sur les récepteurs.

6. Procédé selon la revendication 1, où ladite détermination dudit niveau d'expression de l'interleukine comprend la détection des niveaux d'un gène rapporteur exprimé sous le contrôle de la régulation IL-6 ou IL-8.

7. Procédé selon l'une quelconque des revendications 1 à 6, où un rapport entre les niveaux d'expression de l'interleukine dans la population d'essai et les niveaux d'expression de l'interleukine dans la population témoin d'environ 1,5 ou plus, préférablement de 3,5 ou plus, indique que le composé d'essai est toxique pour les cellules tubulaires proximales rénales.

8. Procédé selon l'une quelconque des revendications 1 à 7, où les cellules tubulaires proximales rénales sont dérivées de cellules somatiques et sont des cellules primaires ou des cellules provenant d'une lignée cellulaire stable.

9. Procédé selon la revendication 8, où les cellules tubulaires proximales rénales sont des cellules tubulaires proximales rénales primaires humaines, des cellules HK-2 ou des cellules LLC-PK1.

10. Procédé selon l'une quelconque des revendications 1 à 7, où les cellules tubulaires proximales rénales sont dérivées de cellules souches et sont différenciées à partir de cellules souches embryonnaires, des cellules souches mésenchymateuses ou de cellules souches pluripotentes induites.

11. Procédé selon l'une quelconque des revendications 1 à 10, où les cellules tubulaires proximales rénales sont des cellules tubulaires proximales rénales humaines.

12. Procédé selon l'une quelconque des revendications 1 à 10, où les cellules tubulaires proximales rénales sont des cellules tubulaires proximales rénales non humaines.

13. Procédé selon l'une quelconque des revendications 1 à 12, où ladite mise en contact est répétée une ou plusieurs fois pendant une période d'environ 3 à environ 14 jours.

14. Procédé selon l'une quelconque des revendications 1 à 13, où ladite mise en contact comprend l'addition du composé d'essai à la population d'essai de cellules tubulaires proximales rénales à une concentration d'environ 0,001 à environ 1000 µg/m1.


### SEQUENCE LISTING

<110> AGENCY FOR SCIENCE, TECHNOLOGY AND RESEARCH
   Zink, Daniele
   Li, Yao
<120> IN VITRO ASSAY FOR PREDICTING RENAL PROXIMAL TUBULAR CELL TOXICITY
<130> 93231-881
<150> US 61/674,018
   <151> 2012-07-20
<150> US 61/675,680
   <151> 2012-07-25
<160> 18
<170> PatentIn version 3.5
<210> 1
   <211> 212
   <212> PRT
   <213> Homo sapiens
<400> 1
   Met Asn Ser Phe Ser Thr Ser Ala Phe Gly Pro Val Ala Phe Ser Leu
   1               5                   10                  15
   Gly Leu Leu Leu Val Leu Pro Ala Ala Phe Pro Ala Pro Val Pro Pro
               20                  25                  30
   Gly Glu Asp Ser Lys Asp Val Ala Ala Pro His Arg Gln Pro Leu Thr
           35                  40                  45
   Ser Ser Glu Arg Ile Asp Lys Gln Ile Arg Tyr Ile Leu Asp Gly Ile
       50                  55                  60
   Ser Ala Leu Arg Lys Glu Thr Cys Asn Lys Ser Asn Met Cys Glu Ser
   65                  70                  75                  80
   Ser Lys Glu Ala Leu Ala Glu Asn Asn Leu Asn Leu Pro Lys Met Ala
                   85                  90                  95
   Glu Lys Asp Gly Cys Phe Gln Ser Gly Phe Asn Glu Glu Thr Cys Leu
               100                 105                 110
   Val Lys Ile Ile Thr Gly Leu Leu Glu Phe Glu Val Tyr Leu Glu Tyr
           115                 120                 125
   Leu Gln Asn Arg Phe Glu Ser Ser Glu Glu Gln Ala Arg Ala Val Gln
       130                 135                 140
   Met Ser Thr Lys Val Leu Ile Gln Phe Leu Gln Lys Lys Ala Lys Asn
   145                 150                 155                 160
   Leu Asp Ala Ile Thr Thr Pro Asp Pro Thr Thr Asn Ala Ser Leu Leu
                   165                 170                 175
   Thr Lys Leu Gln Ala Gln Asn Gln Trp Leu Gln Asp Met Thr Thr His
               180                 185                 190
   Leu Ile Leu Arg Ser Phe Lys Glu Phe Leu Gln Ser Ser Leu Arg Ala
           195                 200                 205
   Leu Arg Gln Met
       210
<210> 2
   <211> 212
   <212> PRT
   <213> Sus scrofa
<400> 2
   Met Asn Ser Leu Ser Thr Ser Ala Phe Ser Pro Val Ala Phe Ser Leu
   1               5                   10                  15
   Gly Leu Leu Leu Val Met Ala Thr Ala Phe Pro Thr Pro Glu Arg Leu
               20                  25                  30
   Glu Glu Asp Ala Lys Gly Asp Ala Thr Ser Asp Lys Met Leu Phe Thr
           35                  40                  45
   Ser Pro Asp Lys Thr Glu Glu Leu Ile Lys Tyr Ile Leu Gly Lys Ile
       50                  55                  60
   Ser Ala Met Arg Lys Glu Met Cys Glu Lys Tyr Glu Lys Cys Glu Asn
   65                  70                  75                  80
   Ser Lys Glu Val Leu Ala Glu Asn Asn Leu Asn Leu Pro Lys Met Ala
                   85                  90                  95
   Glu Lys Asp Gly Cys Phe Gln Ser Gly Phe Asn Gln Glu Thr Cys Leu
               100                 105                 110
   Met Arg Ile Thr Thr Gly Leu Val Glu Phe Gln Ile Tyr Leu Asp Tyr
           115                 120                 125
   Leu Gln Lys Glu Tyr Glu Ser Asn Lys Gly Asn Val Glu Ala Val Gln
       130                 135                 140
   Ile Ser Thr Lys Ala Leu Ile Gln Thr Leu Arg Gln Lys Gly Lys Asn
   145                 150                 155                 160
   Pro Asp Lys Ala Thr Thr Pro Asn Pro Thr Thr Asn Ala Gly Leu Leu
                   165                 170                 175
   Asp Lys Leu Gln Ser Gln Asn Glu Trp Met Lys Asn Thr Lys Ile Ile
               180                 185                 190
   Leu Ile Leu Arg Ser Leu Glu Asp Phe Leu Gln Phe Ser Leu Arg Ala
           195                 200                 205
   Ile Arg Ile Met
       210
<210> 3
   <211> 97
   <212> PRT
   <213> Homo sapiens
<400> 3
   Met Thr Ser Lys Leu Ala Val Ala Leu Leu Ala Ala Phe Leu Ile Ser
   1               5                   10                  15
   Ala Ala Leu Cys Glu Gly Ala Val Leu Pro Arg Ser Ala Lys Glu Leu
               20                  25                  30
   Arg Cys Gln Cys Ile Lys Thr Tyr Ser Lys Pro Phe His Pro Lys Phe
           35                  40                  45
   Ile Lys Glu Leu Arg Val Ile Glu Ser Gly Pro His Cys Ala Asn Thr
       50                  55                  60
   Glu Ile Ile Val Lys Leu Ser Asp Gly Arg Glu Leu Cys Leu Asp Pro
   65                  70                  75                  80
   Lys Glu Asn Trp Val Gln Arg Val Val Glu Lys Phe Leu Lys Arg Ala
                   85                  90                  95
   Glu
<210> 4
   <211> 103
   <212> PRT
   <213> Sus scrofa
<400> 4
   Met Thr Ser Lys Leu Ala Val Ala Phe Leu Ala Val Phe Leu Leu Ser
   1               5                   10                  15
   Ala Ala Leu Cys Glu Ala Ala Val Leu Ala Arg Val Ser Ala Glu Leu
               20                  25                  30
   Arg Cys Gln Cys Ile Asn Thr His Ser Thr Pro Phe His Pro Lys Phe
           35                  40                  45
   Ile Lys Glu Leu Arg Val Ile Glu Ser Gly Pro His Cys Glu Asn Ser
       50                  55                  60
   Glu Ile Ile Val Lys Leu Val Asn Gly Lys Glu Val Cys Leu Asp Pro
   65                  70                  75                  80
   Lys Glu Lys Trp Val Gln Lys Val Val Gln Ile Phe Leu Lys Arg Thr
                   85                  90                  95
   Glu Lys Gln Gln Gln Gln Gln
               100
<210> 5
   <211> 20
   <212> DNA
   <213> artificial
<220>
   <223> synthetic primer
<400> 5
   acctgaggga aactaatctg 20
<210> 6
   <211> 20
   <212> DNA
   <213> artificial
<220>
   <223> synthetic primer
<400> 6
   cgttgataac ctgtccatct 20
<210> 7
   <211> 20
   <212> DNA
   <213> artificial
<220>
   <223> synthetic primer
<400> 7
   caggctgatc ccataatgca 20
<210> 8
   <211> 22
   <212> DNA
   <213> artificial
<220>
   <223> synthetic primer
<400> 8
   ctgcctctcc accaaccttt ac 22
<210> 9
   <211> 23
   <212> DNA
   <213> artificial
<220>
   <223> synthetic primer
<400> 9
   caaggagctg acttcggaac taa 23
<210> 10
   <211> 20
   <212> DNA
   <213> artificial
<220>
   <223> synthetic primer
<400> 10
   tgcactcagc cgtcgataca 20
<210> 11
   <211> 20
   <212> DNA
   <213> artificial
<220>
   <223> synthetic primer
<400> 11
   tggctgcagg acatgacaac 20
<210> 12
   <211> 21
   <212> DNA
   <213> artificial
<220>
   <223> synthetic primer
<400> 12
   tgaggtgccc atgctacatt t 21
<210> 13
   <211> 21
   <212> DNA
   <213> artificial
<220>
   <223> synthetic primer
<400> 13
   ttggcagcct tcctgatttc t 21
<210> 14
   <211> 22
   <212> DNA
   <213> artificial
<220>
   <223> synthetic primer
<400> 14
   gggtggaaag gtttggagta tg 22
<210> 15
   <211> 25
   <212> DNA
   <213> artificial
<220>
   <223> synthetic primer
<400> 15
   gaaccagtag aagacaattg catca 25
<210> 16
   <211> 24
   <212> DNA
   <213> artificial
<220>
   <223> synthetic primer
<400> 16
   ccaggttttc atcatcttca gcta 24
<210> 17
   <211> 23
   <212> DNA
   <213> artificial
<220>
   <223> synthetic primer
<400> 17
   ccccttcatt gacctcaact aca 23
<210> 18
   <211> 19
   <212> DNA
   <213> artificial
<220>
   <223> synthetic primer
<400> 18
   gacggtgcca tggaatttg 19